(19) Europäisches Patentamt
European Patent Office
Office européen des brevets

(11) Publication number: **0 121 117**
**B1**

(12) # EUROPEAN PATENT SPECIFICATION

(45) Date of publication of patent specification: **30.08.89**

(51) Int. Cl.$^4$: **C 07 F 9/65,** A 61 K 31/675

(21) Application number: **84102249.4**

(22) Date of filing: **02.03.84**

(54) **Derivatives of dihydropyridine and production thereof.**

(30) Priority: **04.03.83 JP 36211/83**

(43) Date of publication of application:
**10.10.84 Bulletin 84/41**

(45) Publication of the grant of the patent:
**30.08.89 Bulletin 89/35**

(84) Designated Contracting States:
**CH DE FR IT LI NL SE**

(56) References cited:
**US-A-3 485 847**

**JOURNAL F. PRAKT. CHEMIE, vol. 318, no. 2, 1976, pages 207-220; K. ISSLEIB et al.: "Beiträge zum Reaktionsverhalten der Oxo-alkan-phosphonsäuredialkylester"**

**J. OF GENERAL CHEMISTRY OF THE USSR, vol. 47, no. 5, part 2, 1977, pages 1093-1094, Plenum Publishing Corporation, New York, US; A.I. RAZUMOV et al.: "Phosphorylated dihydropyridines"**

(73) Proprietor: **NIPPON SHINYAKU COMPANY, LIMITED**
**14, Kisshoin Nishinosho Monguchicho**
**Minami-ku Kyoto-shi Kyoto 601 (JP)**

(72) Inventor: **Kimura, Kiyoshi**
**5-55-21 Yasuoka Teramachi**
**Takatsuki-shi Osaka-fu 569 (JP)**
Inventor: **Morita, Iwao**
**28 Koaza Tohokugumi Oazi Tage**
**Ide-cho Tsuzuki-gun Kyoto-fu 610 03 (JP)**
Inventor: **Morimura, Seiichiro**
**405 Ekimae Corporous 294 Katsube-cho**
**Moriyama-shi Shiga-ken 524 (JP)**

(74) Representative: **Wey, Hans-Heinrich, Dipl.-Ing. et al**
**Patentanwälte Wey & Partner**
**Widenmayerstrasse 49**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 121 117 B1**

Courier Press, Leamington Spa, England.

## Description

The present invention relates to 1,4-dihydropyridine-3-carboxylates and salts thereof, to a method of preparing the same, to pharmaceutical compositions containing the same, and to the use of those pharmaceutical compositions in the treatment of angina pectoris and of hypertension.

Compounds of the present invention are represented by the following general formula (I)

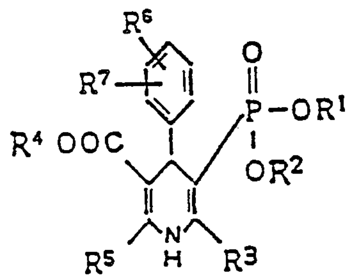

( I )

in which:

$R^1$ and $R^2$ are same or different groups standing for: hydrogen; a hydrocarbyl radical of one to ten carbon atoms containing zero to four unsaturated bonds, and optionally substituted by halogen atom(s) and/or alkoxy group(s); or a tetrahydrofurfuryl group optionally substituted by alkyl group(s);

$R^3$ is lower alkyl group;

$R^4$ is: hydrogen; a hydrocarbyl radical of one to ten carbon atoms containing zero to four unsaturated bonds and optionally substituted by alkoxy, aryloxy, aralkyloxy, amino or alkyl-substituted amino group(s); a group represented by

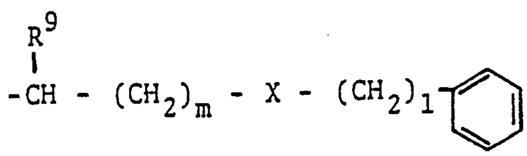

(in which X is $\overset{R^8}{\overset{|}{N}}$ or O; wherein $R^8$ is lower alkyl or lower alkenyl group; $R^9$ is hydrogen, an alkyl group or a phenyl group optionally substituted by alkyl group(s); l is an integer of 0 to 2; m is an integer of 1 to 4); or a group represented by

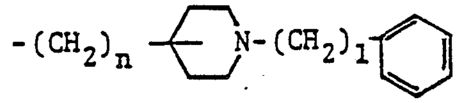

(in which l is the same as already defined; n is an integer of 0 to 2);

$R^5$ is lower alkyl group;

$R^6$ and $R^7$ are same or different groups standing for hydrogen, nitro, cyano, trifluoromethyl, halogen, azido, alkoxycarbonyl, aminocarbonyl, sulfamoyl or alkylsulfonyl or difluoromethoxy, provided that one of $R^6$ and $R^7$ is not hydrogen, and including the following compounds tetrahydrofurfuryl 2,6 - dimethyl - 4 - (3 - nitrophenyl) - 5 - bis(isopropoxy)phosphinyl - 1,4 - dihydropyridine - 3 - carboxylate; 2(methylthio)ethyl 2,6 - dimethyl - 4 - (3 - nitrophenyl) - 5 - bis(isopropoxy)phosphinyl - 1,4 - dihydropyridine - 3 - carboxylate; 3 - pyridylmethyl - 2,6 - dimethyl - 4 - (3 - nitrophenyl) - 5 - bis(isopropoxy)phosphinyl - 1,4 - dihydropyridine - 3 - carboxylate; 2 - (4 - benzyl - 1 - piperazinyl) - ethyl 2,6 - dimethyl - 4 - (3 - nitrophenyl) - 5 - bis(isopropoxy)phosphinyl - 1,4 - dihydropyridine - 3 - carboxylate; 2 - (N - benzyl - N - methylamino) - 1 - (N - benzyl - N - methylaminomethyl) - ethyl 2,6 - dimethyl - 4 - (3 - nitrophenyl) - 5 - bis(isopropoxy)phosphinyl - 1,4 - dihydropyridine - 3 - carboxylat; 3 - pyridylmethyl 2,6 - dimethyl - 4 - (3 - nitrophenyl) - 5 - bis(tetrahydrofurfuryloxy)phosphinyl - 1,4 - dihydropyridine - 3 - carboxylate; and 2 - pyridylmethyl 2,6 - dimethyl - 4 - (3 - nitrophenyl) - 5 - bis(isopropoxy)phosphinyl - 1,4 - dihydropyridine - 3 - carboxylate.

Examples of aliphatic hydrocarbyl radicals in $R^1$, $R^2$ and $R^4$ are methyl, ethyl, n-propyl, isopropyl, n-butyl, isobutyl, secondary butyl, tertiary butyl, n-pentyl, isopentyl, secondary pentyl, neopentyl, tertiary pentyl, 1,2-dimethylpropyl, n-hexyl, isohexyl, 3,3-dimethyl-1-butyl, 1,2,2,-trimethylpropyl, 1-methylpentyl, 1-ethylbutyl, n-heptyl, 1-methylhexyl, 1-ethylpentyl and n-octyl and most preferred are alkyl groups with one to six carbon atoms. Examples of unsaturated hydrocarbonyl radicals are allyl, crotyl, beta-methylallyl, 1-methyl-2-butenyl and 3-methylbut-2-enyl. Examples of cyclic hydrocarbyl radicals are those with three to seven rings. Aromatic groups such as phenyl are included in unsaturated cyclic hydrocarbon groups.

Other examples of $R^1$ and $R^2$ are cycloalkylalkyl groups such as cyclopropylmethyl, cyclobutylmethyl and cyclopentylmethyl.

Other examples of $R^1$, $R^2$ and $R^4$ are alkoxyalkyl groups such as beta-methoxyethyl, beta-ethoxyethyl, beta-n-propoxyethyl, beta-iso-propoxyethyl and 3-methyl-3-methoxybutyl.

Still other examples of $R^1$ and $R^2$ are phenylalkyl groups such as benzyl and phenethyl and phenylalkenyl groups such as cinnamyl.

Still further examples of $R^1$ and $R^2$ are tetrahydrofurfuryl groups.

Examples of alkyl groups in $R^3$ and $R^5$ are lower alkyl groups such as methyl, ethyl and propyl.

Examples of $R^4$ are, besides those already given, aralkyloxyalkyl groups such as beta-benzyloxyethyl and beta-phenethyloxyethyl and aryloxyalkyl groups such as optionally substituted beta-phenoxyethyl groups.

Examples of the group represented by the formula

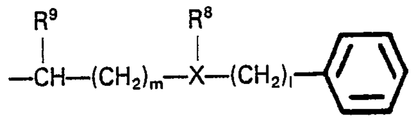

wherein meanings of symbols are the same as already defined are 2-(N-benzyl-N-methylamino)-ethyl, 2-(N-phenethyl-N-methylamino)-ethyl, 3-(N-benzyl-N-methylamino)-propyl, 4-(N-benzyl-N-methylamino)-butyl, 5-(N-benzyl-N-methylamino)-pentyl, 2-(N-benzyl-N-methylamino)-1-phenylethyl and 2-(N-benzyl-N-methylamino)-1-phenylethyl.

Examples of the groups represented by the formula

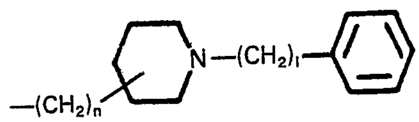

wherein meanings of symbols are the same as already defined are N-benzyl-4-piperidinyl, N-benzyl-3-piperidinyl, N-benzyl-2-piperidinyl, N-phenyl-4-piperidinyl and N-phenethyl-4-piperidinyl.

Examples of the compounds covered by the present invention are, in addition to the compounds listed in examples later in this specification, as follows: methyl 2,6 - dimethyl - 4 - (3 - nitrophenyl) - 5 - di - (1 - methylbutoxy) - phosphinyl - 1,4 - dihydropyridine - 3 - carboxylate; methyl 2,6 - dimethyl - 5 - di - (1,2 - dimethylpropoxy) - phosphinyl - 4 - (3 - nitrophenyl) - 1,4 - dihydropyridine - 3 - carboxylate; methyl 2,6 - dimethyl - 4 - (3 - nitrophenyl) - 5 - di - n - propoxyphosphinyl - 1,4 - dihydropyridinecarboxylate; methyl 2,6 - dimethyl - 5 - [di - (1 - methylpentyloxy) - phosphinyl] - 4 - (3 - nitrophenyl) - 1,4 - dihydropyridine - 3 - carboxylate; methyl - 5 - [di - (1 - ethylbutoxy) - phosphinyl] - 2,6 - dimethyl - 4 - (3 - nitrophenyl) - 1,4 - dihydropyridine - 3 - carboxylate; methyl 2,6 - dimethyl - 4 - (3 - nitrophenyl) - 5 - [di - (2 - isopropoxyethoxy) - phosphinyl] - 1,4 - dihydropyridine - 3 - carboxylate; methyl 5 - dicrotyloxyphosphinyl - 2,6 - dimethyl - 4 - (3 - nitrophenyl) - 1,4 - dihydropyridine - 3 - carboxylate; methyl 5 - dimethallyloxyphosphinyl - 2,6 - dimethyl - 4 - (3 - nitrophenyl) - 1,4 - dihydropyridine - 3 - carboxylate; methyl - 2,6 - dimethyl - 5 - [di - (1 - methyl - 2 - propenyloxy) - phosphinyl] - 4 - (3 - nitrophenyl) - 1,4 - dihydropyridine - 3 - carboxylate; methyl - 2,6 - dimethyl - 4 - (3 - nitrophenyl) - 5 - [di - (3 - methylbut - 2 - enyl) - phosphinoyl] - 1,4 - dihydropyridine - 3 - carboxylate; methyl 5 - dicyclopropylmethyloxyphosphinoyl - 2,6 - dimethyl - 4 - (3 - nitrophenyl) - 1,4 - dihydropyridine - 3 - carboxylate; methyl 2,6 - dimethyl - 4 - (3 - nitrophenyl) - 5 - di - (tetrahydrofurfuryloxy) - phosphinyl - 1,4 - dihydropyridine - 3 - carboxylate; 2 - (dimethylamino)ethyl 5 - dimethoxyphosphinyl - 2,6 - dimethyl - 4 - (3 - nitrophenyl) - 1,4 - dihydropyridine - 3 - carboxylate; 2 - propoxyethyl 5 - dimethoxyphosphinyl - 2,6 - dimethyl - 4 - (3 - nitrophenyl) - 1,4 - dihydropyridine - 3 - carboxylate; 2 - (dimethylamino)ethyl 5 - diethoxyphosphinyl - 2,6 - dimethyl - 4 - (3 - nitrophenyl) - 1,4 - dihydropyridine - 3 - carboxylate; 2 - n - propoxyethyl 5 - diethoxyphosphinyl - 2,6 - dimethyl - 4 - (3 - nitrophenyl) - 1,4 - dihydropyridine - 3 - carboxylate; 2 - (N - benzyl - N - methylamino) - ethyl 2,6 - dimethyl - 4 - (3 - nitrophenyl) - 5 - [di - (1 - methylbutoxy) - phosphinyl] - 1,4 - dihydropyridine - 3 - carboxylate; 2 - (N - benzyl - N - methylamino) - ethyl - 2,6 - dimethyl - 5 - [di - (1,2 - dimethylpropoxy) - phosphinyl] - 4 - (3 - nitrophenyl) - 1,4 - dihydropyridine - 3 - carboxylate; 2 - (N - benzyl - N - methylamino) - ethyl - 2,6 - dimethyl - 4 - (3 - nitrophenyl) - 5 - di - n - pentyloxyphosphinyl - 1,4 - dihydropyridine - 3 - carboxylate; 2 - (N - benzyl - N - methylamino) - ethyl 2,6 - dimethyl - 5 - [di - (1 - methylpentyloxy) - phosphinyl] - 4 - (3 - nitrophenyl) - 1,4 - dihydropyridine - 3 - carboxylate; 2 - (N - benzyl - N - methylamino) - ethyl - 5 - [di - (1 - ethylbutoxy) - phosphinyl] - 2,6 - dimethyl - 4 - (3 - nitrophenyl) - 1,4 - dihydropyridine - 3 - carboxylate; ethyl 5 - [di - (2 - methoxyethoxy) - phosphinyl] - 2,6 - dimethyl - 4 - (3 - nitrophenyl) - 1,4 - dihydropyridine - 3 - carboxylate; isopropyl 5 - [di - (2 - methoxyethoxy) - phosphinyl] - 2,6 - dimethyl - 4 - (3 - nitrophenyl) - 1,4 - dihydropyridine - 3 - carboxylate; 2 - (dimethylamino)ethyl 5 - [di - (2 - methoxyethoxy) - phosphinyl] - 2,6 - dimethyl - 4 - (3 - nitrophenyl) - 1,4 - dihydropyridine - 3 - carboxylate; 2 - n - propoxyethyl 5 - [di - (2 - methoxyethoxy) - phosphinyl] - 2,6 - dimethyl - 4 - (3 - nitrophenyl) - 1,4 - dihydropyridine - 3 -

carboxylate; N - benzyl - 4 - piperidinyl 5 - [di - (2 - methoxyethoxy) - phosphinyl] - 2,6 - dimethyl - 4 - (3 - nitrophenyl) - 1,4 - dihydropyridine - 3 - carboxylate; 2 - n - propoxyethyl 5 - [di - (2 - ethoxyethoxy) - phosphinyl] - 2,6 - dimethyl - 4 - (3 - nitrophenyl) - 1,4 - dihydropyridine - 3 - carboxylate; 2 - (N - benzyl - N - methylamino) - ethyl - 2,6 - dimethyl - 4 - (3 - nitrophenyl) - 5 - (di - (2 - isopropoxyethoxy) - phosphinyl] - 1,4 - dihydropyridine - 3 - carboxylate; 2 - (dimethylamino)ethyl 5 - diallyloxyphosphinyl - 2,6 - dimethyl - 4 - (3 - nitrophenyl) - 1,4 - dihydropyridine - 3 - carboxylate; 2 - n - propoxyethyl 5 - diallyloxyphosphinyl - 2,6 - dimethyl - 4 - (3 - nitrophenyl) - 1,4 - dihydropyridine - 3 - carboxylate; 2 - (N - benzyl - N - methylamino) - ethyl 5 - dicrotyloxyphosphinyl - 2,6 - dimethyl - 4 - (3 - nitrophenyl) - 1,4 - dihydropyridine - 3 - carboxylate; 2 - (N - benzyl - N - methylamino) - ethyl 5 - dimethallyloxyphosphinyl - 2,6 - dimethyl - 4 - (3 - nitrophenyl) - 1,4 - dihydropyridine - 3 - carboxylate; 2 - (N - benzyl - N - methylamino) - ethyl 5 - dicyclopropylmethoxyphosphinyl - 2,6 - dimethyl - 4 - (3 - nitrophenyl) - 1,4 - dihydropyridine - 3 - carboxylate; 2 - (N - benzyl - N - methylamino) - ethyl 2,6 - dimethyl - 4 - (3 - nitrophenyl) - 5 - [di - (tetrahydrofurfuryloxy) - phosphinyl] - 1,4 - dihydropyridine - 3 - carboxylate; methyl 5 - dimethoxyphosphinyl - 2,6 - dimethyl - 4 - (2 - nitrophenyl) - 1,4 - dihydropyridine - 3 - carboxylate; 2 - (N - benzyl - N - methylamino) - ethyl 5 - dimethoxyphosphinyl - 2,6 - dimethyl - 4 - (2 - nitrophenyl) - 1,4 - dihydropyridine - 3 - carboxylate; methyl 5 - [di - (2 - methoxyethoxy) - phosphinyl] - 2,6 - dimethyl - 4 - (2 - nitrophenyl) - 1,4 - dihydropyridine - 3 - carboxylate; 2 - (N - benzyl - N - methylamino) - ethyl 5 - [di - (2 - methoxyethoxy) - phosphinyl] - 2,6 - dimethyl - 4 - (2 - nitrophenyl) - 1,4 - dihydropyridine - 3 - carboxylate; methyl 5 - diallyloxyphosphinyl - 2,6 - dimethyl - 4 - (2 - nitrophenyl) - 1,4 - dihydropyridine - 3 - carboxylate; 2 - (N - benzyl - N - methylamino) - ethyl 5 - diallyloxyphosphinyl - 2,6 - dimethyl - 4 - (2 - nitrophenyl) - 1,4 - dihydropyridine - 3 - carboxylate; methyl 5 - di - (2 - methoxyethoxy) - phosphinyl - 2,6 - dimethyl - 4 - (2 - trifluoromethylphenyl) - 1,4 - dihydropyridine - 3 - carboxylate; 2 - (N - benzyl - N - methylamino) - ethyl 5 - [di - (2 - methoxyethoxy) - phosphinyl] - 2,6 - dimethyl - 4 - (2 - trifluoromethylphenyl) - 1,4 - dihydropyridine - 3 - carboxylate; methyl 5 - diallyloxyphosphinyl - 2,6 - dimethyl - 4 - (2 - trifluoromethylphenyl) - 1,4 - dihydropyridine - 3 - carboxylate; 2 - (N - benzyl - N - methylamino) - ethyl 5 - diallyloxyphosphinyl - 2,6 - dimethyl - 4 - (2 - trifluoromethyl) - phenyl) - 1,4 - dihydropyridine - 3 - carboxylate; methyl 5 - [di - (2 - methoxyethoxy) - phosphinyl] - 2,6 - dimethyl - 4 - (3 - trifluoromethylphenyl) - 1,4 - dihydropyridine - 3 - carboxylate; 2 - (N - benzyl - N - methylamino) - ethyl 5 - [di - (2 - methoxyethoxy) - phosphinyl] - 2,6 - dimethyl - 4 - (3 - trifluoromethylphenyl) - 1,4 - dihydropyridine - 3 - carboxylate; methyl 5 - diallyloxyphosphinyl - 2,6 - dimethyl - 4 - (3 - trifluoromethylphenyl) - 1,4 - dihydropyridine - 3 - carboxylate; and 2 - (N - benzyl - N - methylamino) - ethyl 5 - diallyloxyphosphinyl - 2,6 - dimethyl - 4 - (3 - trifluoromethylphenyl) - 1,4 - dihydropyridine - 3 - carboxylate.

The compounds of the present invention have asymmetric carbon atom(s) and all of optically active compounds and mixtures thereof are within a scope of the present invention. Racemic compounds may, if desired, be separated by treating with an optically active acid when the present invention compound is a base and the optically active base can be obtained from the resulting salt. When the present invention compound is an acid, an optically active acid can be similarly obtained by the treatment with optically active base.

The compounds of the present invention exhibit vasodilating, peripheral blood vessel dilating and blood pressure decreasing action and are useful for the treatment of angina pectoris, hypertension (high blood pressure), brain circulatory disorder, and other circulatory diseases.

Among derivatives of 1,4-dihydropyridine, dimethyl 2,6-dimethyl-4-(2-nitrophenyl)-1,4-dihydropyridine-3,5-dicarboxylate (generic name: nifedipine) has been known as a compound exhibiting coronary dilating and hypotensive action. The present inventors have investigated novel compounds exhibiting coronary dilating and hypotensive action and, as a reuslt, they have found that novel 1,4-dihydropyridine-3-carboxylic acids, derivatives of the formula (I) and salts thereof show marked vasodilative and hypotensive action with high safety.

In the field of derivatives represented by the formula (I), the following dihydropyridine compounds are known. (cf. von K. Issleid, R. Wolff und M. Lengies: J. prakt. Chemie, 318, pages 207—220, 1976).

In the report by Issleib, et al, the preparation of only two compounds as given below is disclosed. As stated later, these two compounds do not exhibit hypotensive action.

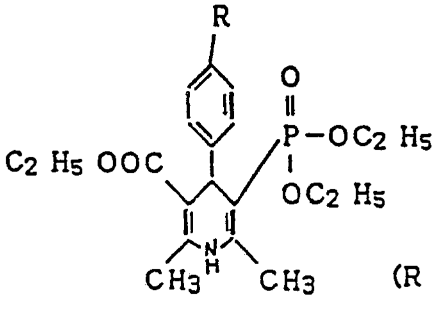

( VI )

(R = H, $OCH_3$)

An object of the present invention is that, by introducing various electro-negative groups into phenyl group substituted at 4-position of 1,4-dihydropyridine, novel and excellent derivatives exhibiting marked vasodilating and hypotensive action are produced.

Main preparation routes for the synthesis of the present invention compounds (I) are exemplified as follows.

Preparation Method 1

( IV ) ( II ) ( III-N )

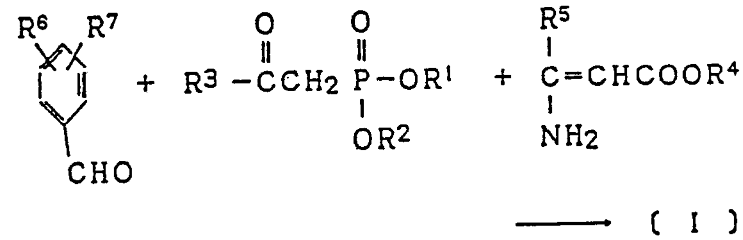

( I )

(in which meanings of $R^1$, $R^2$, $R^3$, $R^4$, $R^5$, $R^6$ and $R^7$ are the same as already defined).

(III-N) means enaminoester of (III). Now, the starting materials (IV), (II) and (III-N) are mixed, preferably in a molar ratio of 1:0.8:0.8 to 1:4:4 and, more preferably, in a molar ratio of 1:0.9:0.9 to 1:1.5:1.5. The reaction is conducted at room temperature to 150°C, more preferably 40 to 100°C, in the presence or absence of alcohols such as methanol, ethanol, isopropanol, aromatic hydrocarbons such as benzene, toluene, halogenated hydrocarbons such as chloroform, carbon tetrachloride, ethers such as tetrahydrofuran, dioxane, non-protonic solvents such as acetonitrile, dimethylformamide, or water.

Separation of desired compound from the reaction mixture is carried out by conventional methods such as, for example, concentration, extraction, column chromatography and recrystallization.

Other preparation methods are exemplified hereinafter with reaction formulas. Those preparation methods may be conducted by or with the same molar ratio, reaction solvent, reaction temperature, separation methods.

Preparation Method 2

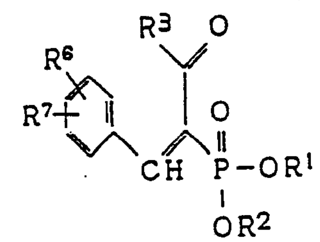

+ ( III-N ) ( I )

( V )

(in which meanings of $R^1$, $R^2$, $R^3$, $R^4$, $R^5$, $R^6$ and $R^7$ are the same as already defined).

The compound (V) used as a starting material can easily be obtained by a condensation of (IV) and (II) used in the preparation method 1 in an organic solvent.

Preparation Method 3

(V) +

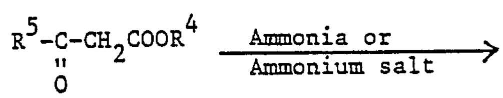


(I)

(III)

Preparation Method 4

( VI ) +

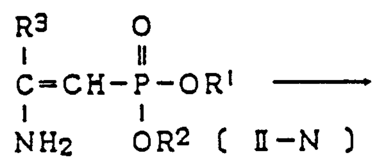

( II-N ) ( I )

(in which meanings of $R^1$, $R^2$, $R^3$, $R^4$, $R^5$, $R^6$ and $R^7$ are the same as already defined).

Preparation Method 5

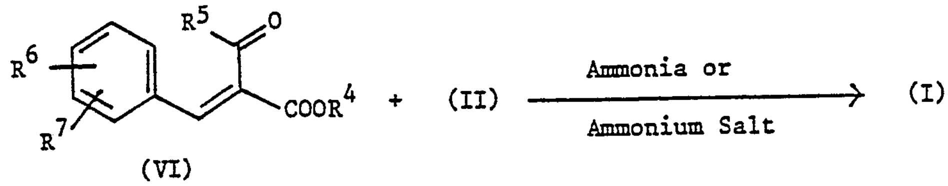

(VI)

Preparation Method 6

$$(IV) + (II) + (III) \xrightarrow[\text{Ammonium Salt}]{\text{Ammonia or}} (I)$$

Ammonium salts hereinabove include ammonium acetate, ammonium carbonate and ammonium bicarbonate.

Phosphonic acid esters represented by a formula (II) used as a starting material are known in the literature or can be prepared by methods known in the literature such as, for example, A. N. Pudovik and V. P. Aver'yanoura, Zhur. Obshch. Doklady Akad. Nauk S. S. S. R., 101, pages 889—892 (1955).

beta-Ketocarboxylic acid esters represented by a formula (III) used as a starting material are known in the literature or can be prepared by methods known in the literaure such as, for example, D. Borrmann "Umsetzung von Diketen mit Alkoholen, Phenolen und Mercaptanen" in Houben-Weyl, Methoden der organischen Chemie, Vol. VII/4, page 230 (1968); Y. Oikawa, K. Sugano and O. Yonemitsu, J. Org. Chem., 43, page 2087 (1978).

Enaminoesters represented by a formula (III-N) used as a starting material are known in the literature or can be prepared by methods known in the literature such as, for example, A. C. Cope, et al., J. Am. Chem. Soc., 67, page 1017 (1945).

Enaminoesters of a formula (II-N) can also be prepared by the same methods as for (III-N).

Aromatic aldehydes (IV) used as a starting material are known in the literature or can be prepared by methods known in the literature such as, for example, E. Mosettig, Organic Reactions, VII, pages 218 et seq. (1954).

Benzylidene derivatives (V) used as a starting material are prepared by known methods in the literature such as, for example, A. N. Pudoruk, G. E. Yastrebova and V. I. Nikitina, Zh. Obshch. Khim. 37, pages 510—511 (1967) and R. Wolff und M. Lengies, J. prakt. Chem. 318, pages 207—220 (1976). Examples are given in References Examples disclosed later.

Benzylidene derivatives (VI) used as a starting material are known in literatures or can be prepared by known methods in the literature such as, for example, G. Jones, "The Knoevenagel Condensation" in Organic Reactions, Vol. XV, pages 204 et seq (1967).

Now, the preparation methods for starting materials for the present invention compounds (I) are illustrated in detail. However, the present invention is not limited thereto.

Reference Example

To 300 ml of toluene were added 38.2 grams of dimethyl acetonylphosphonate and *m*-nitrobenzaldehyde, then 1.96 grams of piperidine and 2 ml of acetic acid were added thereto, and the mixture was heated to reflux for 16 hours with stirring. The reaction was conducted using a water remover so that water produced thereby was taken away. After cooled, the reaction solution was washed with 5% sodium hydroxide solution, then with 20% sodium bisulfite solution, and finally with water. Then it was dried, toluene was evaporated therefrom, and the residue was purified by subjecting to a silica gel column chromatograpy (350 grams of silica gel being used; eluting solution was a mixture of n-hexane and ethyl acetate) to afford 42.6 grams of dimethyl 1-(3-nitrobenzylidene)-acetonylphosphonate, pale brown oil, yield 65%. IR spectra (film, $cm^{-1}$): 1705, 1615, 1535, 1360, 1265, 1060, 1035, 820. NMR spectra ($CDCl_3$, δ): 2.30 (3H, s); 3.72 (6H, d, J=11Hz), 7.3—7.8 (3H, m), 8.0—8.3 (2H, m).

The following compounds were also manufactured by the same way as in the Reference Example 1.

Diethyl 1-(3-nitrobenzylidene)-acetonylphosphonate. Pale yellowish brown oil. yield 65%. IR (film, $cm^{-1}$): 1705, 1615, 1535, 1355, 1260.

Di-isopropyl 1-(3-nitrobenzylidene)-acetonylphosphonate. Pale brown oil. Yield 60%. IR (film, $cm^{-1}$): 1705, 1615, 1535, 1355, 1260.

n-Propyl 1-(3-nitrobenzylidene)-acetonylphosphonate. Pale brown oil. Yield 71%. IR (film, $cm^{-1}$): 1705, 1615, 1535, 1355, 1260.

Di-n-butyl 1-(3-nitrobenzylidene)-acetonylphosphonate. Pale brown oil. Yield 62%. IR (film, $cm^{-1}$): 1710, 1615, 1535, 1360, 1255.

Diisobutyl 1-(3-nitrobenzylidene)-acetonylphosphonate. Pale yellow crystals, melting point 81—82°C (ether). Yield 57%. IR (KBr, $cm^{-1}$): 1705, 1615, 1535, 1355, 1255.

Di-sec-butyl 1-(3-nitrobenzylidene)-acetonylphosphonate. Pale brown oil. Yield 58%. IR (film, $cm^{-1}$): 1710, 1620, 1535, 1360, 1255.

Diisopentyl 1-(3-nitrobenzylidene)-acetonylphosphonate. Pale brown oil. Yield 55%. IR (film, $cm^{-1}$): 1705, 1615, 1530, 1350, 1260.

Di-n-pentyl 1-(3-nitrobenzylidene)-acetonylphosphonate. Pale brown oil. Yield 48%. IR (film, $cm^{-1}$): 1710, 1615, 1530, 1350, 1260.

Di-(1-methylbutyl) 1-(3-nitrobenzylidene)-acetonylphosphonate. Pale brown oil. Yield 56%. IR (film, $cm^{-1}$): 1710, 1610, 1530, 1350, 1255.

Dineopentyl 1-(3-nitrobenzylidene)-acetonylphosphonate. Pale yellow crystals, melting point 139—140°C (ether). Yield 50%. IR (KBr, $cm^{-1}$): 1710, 1610, 1530, 1350, 1250.

Dicyclopentyl 1-(3-nitrobenzylidene)-acetonylphosphonate. Pale brown oil. Yield 52%. IR (film, $cm^{-1}$): 1710, 1615, 1535, 1355, 1260.

Diphenyl 1-(3-nitrobenzylidene)-acetonylphosphonate. Pale yellow crystals, melting point 108—111°C (ether). Yield 45%. IR (film, $cm^{-1}$): 1710, 1615, 1535, 1355, 1255.

Di-2-methoxyethyl 1-(3-nitrobenzylidene)-acetonylphosphonate. Pale yellow oil. Yield 44%. IR (film, $cm^{-1}$): 1700, 1610, 1530, 1350, 1255.

Diallyl 1-(3-nitrobenzylidene)-acetonylphosphonate. Pale yellow oil, Yield 62%. IR (film, $cm^{-1}$): 1705, 1615, 1535, 1355, 1250, 1050, 1000.

Diisopropyl 1-(2-chlorobenzylidene)-acetonylphosphonate. Pale yellow oil. IR (film, $cm^{-1}$): 1705, 1615, 1260, 1055, 1000, 990.

Diisopropyl 1-(3-chlorobenzylidene)-acetonylphosphonate. Pale yellow oil. IR (film, $cm^{-1}$): 1700, 1610, 1570, 1255, 1050, 1000.

Diisopropyl 1-(2-cyanobenzylidene)-acetonylphosphonate. Pale yellow oil. IR (film, $cm^{-1}$): 2240, 1700, 1620, 1595, 1250, 1050, 1040, 1000, 990.

Diisopropyl 1-(2-trifluoromethylbenzylidene)-acetonylphosphonate. Pale yellow oil. IR (film, $cm^{-1}$): 1710, 1615, 1580, 1320, 1260, 1175, 1120, 1050, 1035, 980.

Diisopropyl 1-(3-trifluoromethylbenzylidene)-acetonylphosphonate. Pale yellow oil. IR (film, $cm^{-1}$): 1710, 1615, 1335, 1260, 1130, 1000.

The compounds of the present invention and pharmaceutically acceptable salts thereof exhibit strong coronary dilating, hypotensive, and other vasodilating action and, since they have a low toxicity, it is expected that the compounds will be widely applicable for the treatment of diseases of the circulation system such as, for example, hypertension, heart disorder, arrythmias, angina pectoris, myocardial infarction, brain blood vein disturbance, and peripheral circulatory disturbance.

Representative examples of the present invention compounds and pharmaceutically acceptable salts thereof were tested as to their effect to coronary artery and other blood vessels and blood pressure and compared with those of diltiazem, a known compound, and the results are given below.

1. Test Method and Result.

a) Coronary dilating action.

In accordance with Langendorff's method, the effect of the compounds of the present invention given by intracoronary administration to a constantly perfused isolated guinea pig heart preparation was examined. The potency of coronary dilating action was evaluated from the decreasing rate of perfusion pressure under each dose ($10^{-7}$, $10^{-6}$, and $10^{-5}$ grams/heart) of the compounds of the present invention. The results are given in Table 1.

TABLE 1

| Compounds Tested (Example Number) | Coronary Dilating Action (%) $10^{-7}$ | $10^{-6}$ | $10^{-5}$ gram/heart |
|---|---|---|---|
| 1 | 7.2 | 16.0 | 26.9 |
| 2 | 5.0 | 18.7 | 41.9 |
| 3 | 12.8 | 27.9 | 36.0 |
| 4 | 21.7 | 32.0 | 35.6 |
| 5 | 23.3 | 36.2 | 40.4 |
| 6 | 8.4 | 18.1 | 31.5 |
| 7 | 17.0 | 20.6 | 28.3 |
| 9 | 12.0 | 25.2 | 40.0 |

TABLE 1 (continued)

| Compounds Tested (Example Number) | Coronary Dilating Action (%) $10^{-7}$ | $10^{-6}$ | $10^{-5}$ gram/heart |
|---|---|---|---|
| 11 | 16.5 | 27.5 | 30.4 |
| 15 | 7.2 | 20.4 | 38.0 |
| 16 | 8.1 | 13.0 | 24.3 |
| 18 | 8.0 | 12.5 | 26.5 |
| 19 | 23.1 | 26.2 | 38.6 |
| 20a | 10.8 | 21.3 | 33.1 |
| 20b | 9.0 | 18.1 | 22.1 |
| 22 | 14.0 | 19.0 | 29.0 |
| 27 | 20.5 | 27.0 | 32.5 |
| 28 | 16.6 | 25.3 | 43.8 |
| 34 | 31.3 | 26.5 | 38.3 |
| 39 | 16.5 | 25.5 | 29.2 |
| 41 | 3.7 | 18.8 | 33.2 |
| 44 | 9.4 | 16.2 | 28.3 |
| 45 | 10.2 | 27.5 | 31.5 |
| 47 | 8.1 | 13.3 | 28.5 |
| 48 | 25.3 | 34.5 | 35.7 |
| 50 | 12.0 | 24.4 | 31.1 |
| 52 | 18.1 | 28.0 | 37.0 |
| 60 | 11.5 | 19.2 | 38.0 |
| 62 | 17.8 | 23.1 | 42.6 |
| 63 | 18.9 | 17.0 | 30.7 |
| 65 | 30.2 | 41.5 | 45.7 |
| 82 | 30.6 | 38.3 | 47.2 |
| 91 | 36.7 | 50.3 | 63.2 |
| 99 | 16.1 | 20.9 | 38.3 |
| 131 | 11.3 | 13.4 | 28.8 |
| 145 | 14.2 | 32.0 | 43.6 |
| 149 | 16.0 | 34.9 | 52.8 |
| 153 | 10.7 | 23.7 | 41.6 |

TABLE 1 (continued)

| Compounds Tested (Example Number) | Coronary Dilating Action (%) $10^{-7}$ | $10^{-6}$ | $10^{-5}$ gram/heart |
|---|---|---|---|
| 154 | 2.3 | 31.7 | 52.6 |
| 156 | 15.4 | 22.2 | 35.8 |
| 159 | 62.0 | 72.0 | 74.3 |
| 160 | 15.4 | 30.4 | 39.8 |
| 162 | 26.9 | 29.3 | 33.2 |
| 163 | 12.0 | 38.7 | 44.9 |
| 166 | 16.2 | 32.8 | 53.2 |
| 168 | 10.5 | 61.0 | 68.3 |
| 170 | 37.1 | 48.3 | 59.6 |
| 173 | 23.1 | 35.3 | 47.7 |
| 174 | 10.9 | 39.5 | 51.0 |
| 177 | 17.4 | 18.1 | 28.0 |
| 178 | 10.2 | 36.1 | 43.0 |
| 179 | 28.4 | 61.3 | 66.3 |
| Diltiazem | 10.2 | 28,3 | 35,7 |

b) Hypotensive Action.

Normal rats were used. Under unaesthetized condition, blood pressure of the femoral artery was measured by a pressure transducer and hypotensive action of the compounds of the present invention was evaluated as follows.

Thus, the compounds of the present invention were administered per os at various doses (i.e. 3, 10, and 30 mg/kg) and the variation of the blood pressure with time at each dose was observed. Then the maximum rate of decrease of blood pressure, blood pressure decreasing rate as a (%) of the mean blood pressure in each group was used for evaluation. The results are given in Table 2.

At the same time, the same test was carried out with the two compounds represented by the formula (VII) (R = hydrogen and $OCH_3$) but neither of them showed any hypotensive action at a dose of 30 mg/kg.

TABLE 2

| Compounds Tested (Example Number) | Hypotensive Action (%) 3 | 10 | 30 mg/kg |
|---|---|---|---|
| 2 | 10.9 | 16.6 | 31.7 |
| 5 | 23.5 | 30.1 | 49.2 |
| 11 | 12.2 | 17.6 | 36.1 |
| 15 | 6.5 | 11.0 | 22.4 |
| 19 | 13.0 | 22.8 | 43.0 |
| 20a | 7.2 | 8.3 | 20.0 |

TABLE 2 (continued)

| Compounds Tested (Example Number) | Hypotensive Action (%) 3 | 10 | 30 mg/kg |
|---|---|---|---|
| 22 | 8.1 | 26.0 | 38.6 |
| 27 | 12.5 | 36.7 | 49.0 |
| 34 | 21.2 | 33.7 | 50.4 |
| 44 | 12.7 | 18.8 | 37.9 |
| 46 | 18.3 | 24.2 | 39.8 |
| 48 | 24.1 | 47.6 | 48.3 |
| 50 | 18.0 | 27.1 | 50.0 |
| 60 | 20.6 | 28.5 | 50.1 |
| 62 | 18.3 | 32.0 | 45.6 |
| 63 | 12.1 | 14.2 | 33.3 |
| 65 | 13.2 | 20.9 | 47.8 |
| 79 | 13.2 | 44.5 | 33.2 |
| 82 | 37.2 | 38.1 | 46.0 |
| 86 | 11.9 | 27.4 | 34.1 |
| 89 | 5.3 | 28.2 | 49.6 |
| 90 | 20.2 | 26.4 | 36.8 |
| 91 | 15.1 | 43.3 | 38.7 |
| 94 | 8.5 | 23.1 | 38.7 |
| 99 | 28.5 | 44.4 | 45.7 |
| 100 | 3.8 | 16.5 | 33.6 |
| 131 | 17.2 | 25.5 | 42.3 |
| 145 | 12.6 | 24.5 | 48.9 |
| 151 | 16.8 | 27.5 | 43.3 |
| 162 | 2.8 | 34.9 | 51.9 |
| 164 | 21.3 | 37.7 | 39.7 |
| 166 | 3.1 | 34.4 | 44.5 |
| 170 | 10.3 | 29.7 | 34.2 |
| 179 | 12.9 | 37.4 | 47.8 |
| Diltiazem | — | 25,0 | 33,2 |

c) Acute Toxicity.

The compounds of the present invention exhibit little toxicity and their $LD_{50}$ values by oral administration to rats are above 400 mg/kg.

When the compounds of the present invention are administered as drugs to animals including human being, they can be given directly or as pharmaceutical composition in pharmaceutically acceptable non-toxic and inert carriers within a range of, for example, 0.1% to 99.5% and, more preferably, 0.5% to 90%.

Examples of the carriers applicable are solid, semi-solid or liquid diluents, fillers and other auxiliaries for pharmaceutical compositions and a mixture thereof. It is desired that the pharmaceutical compositions are administered in a unit dose form. The pharmaceutical compositions of the present invention can be administered orally, interstitially, locally (such as percutanously), or rectally. Administration is, of course, to be conducted using pharmaceutical dosage forms suitable for each route. For example, injections are particularly preferred.

It is desired that the dose is regulated by taking the status of patients (such as age, body weight, etc), administration route, and the kind and degree of the disease into consideration. Generally it is within a range of 1 to 1000 mg of the effective constituent per day. In some cases, it is sufficient to use lower doses than above and, in some other cases, it will be necesary to use higher doses. When a large amount is given, it is recommended to divide the daily dose and administer it several times a day.

Oral administration is carried out by solid or liquid unit dose forms such as, for example, pure powder, diluted powder, tablets, sugar coated tablets, capsules, granules, suspensions, liquids, syrups and sublingual tablets.

Pure powder is manufactured by pulverizing the active substance into a suitable size. Diluted powder is manufactured by pulverizing the active substances into a suitable size followed by mixing it with similarly pulverized pharmaceutical carriers such as, for example, edible carbohydrates (starch, mannitol). If necessary, flavouring agents, preservatives, dispersing agents, colouring agents, and perfumes, may be added thereto.

Capsules are manufactured by filling the above powdery substance (pure powder or diluted powder) or granules which are fully described under tablets into capsules such as gelatin capsules. It is possible to mix the powder with lubricants or fluidizing agents such as, for example, colloidal silica, talc, magnesium stearate, calcium stearate, solid polyethylene glycol, and then filling capsules with the mixture. When disintegrating agents and solubilizers such as, for example, carboxymethylcellulose, calcium carboxymethylcellulose, lower substituted hydroxy, propylcellulose calcium carbonate, sodium carbonate, are added thereto, it is possible to improve the effectiveness of pharmaceuticals when the capsules are administered.

It is also possible to suspend the powder and/or disperse in vegetable oils, polyethylene glycol, glycerol, or surface active agents, then put the suspension and/or dispersion in gelatine sheets to prepare soft capsules.

Tablets are manufactured by first preparing a powder mixture, then converting it into granules or slugs, followed by adding disintegrating agents or lubricants thereto, and finally by compressing the grnaules or slugs into tablets.

Mixed powder is manufactured by mixing suitably pulverized substance with above-mentioned diluents or bases and, if necessary, with binders (such as sodium carboxymethylcellulose, alginates, gelatine, polyvinylpyrrolidone, polyvinyl alcohol), solubilization retarding agents (such as paraffin), reabsorbing agents (such as quaternary salts) and/or adsorbing agents (such as bentonite, kaoline, dicalcium phosphate). Mixed powder is first wetted with a binder solution (such as syrup, starch paste, gum arabicum, cellulose solution) or with polymer solution and then forced through sieves to afford granules. Instead of preparing granules from the powder as mentioned above, it is possible to manufacture granules by passing the mixed powder through tableting machines and then by pulverizing the resulting slugs of nonuniform sizes.

To the resulting granules is added a lubricant such as, for example, stearic acid, stearates, talc or mineral oil, so that sticking of granules to each other can be prevented. Such lubricated mixtures are then made into tablets. It is also possible that the pharmaceuticals may, instead of being converted into granules or slugs as mentioned hereinabove, be mixed with inert carriers having fluidity and then directly made into tablets. Transparent or semitransparent protective membranes consisting of closed membrane of shellac, a membrane composed of sugars or polymers, and brushing coatings composed of wax may also be applied.

Other orally administrable dosage forms such as, for example, solutions, syrups, and elixiers can also be made into unit dosage forms in which a determined amount of the drug is contained therein. Syrups are manufactured by dissolving a compound in a suitable flavoured solution and elixiers are manufactured by the use of non-toxic alcoholic carriers. Suspensions are manufactured by dispersing the compound in non-toxic carriers. Solubilizers and emulsifiers (such as ethoxylated isostearyl alcohols, polyoxyethylene sorbitol esters), preservatives and flavouring agents (such as peppermint oil, saccharine) may also be added thereto.

If necessary, unit dosage compositions for oral administration may be in a form of microcapsules. Said compositions may also be coated or embedded in polymers or waxes so that prolongation of the duration of action or sustained release of the active ingredient can be achieved.

Parenteral administration including subcutaneous intramuscular or intravenous injections can be carried out using liquid unit dosage forms such as in a form of solution or suspension. They are manufactured by dissolving or suspending a definite amount of the compound in an aqueous or oily solvent or other non-toxic liquidal carriers suitable for the purpose of injection followed by sterilizing the said suspension or solution. Alternatively, a definite amount of the compound is placed in a vial and the vial as well as the content therein may be sterilized and sealed. In order to dissolve or mix immediately before administration, it is recommended to prepare auxiliary vials or carriers in addition to powdered or lyophilized active constituents. In order to make the injection solution isotonic, it is suggested to add a non-toxic salt or solution thereof. It is further possible to add stabilizers, preservatives and emulsifiers.

Rectal administration can be carried out using suppositories which are prepared by mixing the compound with low-melting and water soluble or insoluble solids such as, for example, polyethylene glycol, cacao butter, higher esters (such as myristyl palmitate), and mixtures thereof.

To the pharmaceutical preparations of the present invention other pharmaceutical compounds such as nitrites, beta-blockers and diuretic hypotensive drugs may be added. These may also be simultaneously given to patients together with the compositions of the invention.

As hereunder, preparation of the compounds of the present invention is exemplified but it is to be understood that the present invention is not limited thereto.

Example 1

To a mixture of 1.85 grams of dimethyl 1-(3-nitrobenzylidene)acetonylphosphonate and 0.75 gram of methyl 3-aminocrotonate was added 25 ml of isopropanol and the mixture was heated to reflux for four hours with stirring. The reaction mixture was concentrated in vacuo, to the residue was added ether, the resulting crystals were collected by filtration, and recrystallized from ethyl acetate to afford 1.08 grams (yield 42%) of methyl 5-dimethoxyphosphinyl-2,6-dimethyl-4-(3-nitrophenyl)-1,4-dihydropyridine-3-carboxylate, pale yellow crystals, melting point 221—3°C.

IR spectra (KBr, $cm^{-1}$): 3300, 3240, 1708, 1650, 1535, 1355.

NMR spectra ($CDCl_3$, δ): 2.31 (3H, d, J=2.5Hz) 2.36 (3H, s) 3.35 (3H, d, J=11Hz) 3.54 (3H, d, J=10Hz) 3.65 (3H, s) 4.80 (1H, d, J=10Hz) 6.01 (1H, d, J=5Hz), 7.37 (1H, t, J=8Hz) 7.61—7.68 (1H, m) 7.96—8.04 (1H, m) 8.11 (1H, t, J=2Hz).

Elementary analysis for: $C_{17}H_{21}N_2O_7P$

Calculated (%): C: 51.52 H: 5.34 N: 7.07

Determined (%): C: 51.48 H: 5.57 N: 6.98

Example 2

To a mixture of 3.42 grams of dimethyl 1-(3-nitrobenzylidene)acetonylphosphonate and 3.13 grams of 2-(N-benzyl-N-methylamino)ethyl 3-aminocrotonate was added 50 ml of isopropanol and the mixture was heated to reflux for four hours with stirring. The reaction mixture was concentrated in vacuo, the residue was dissolved in ethyl acetate, to this was added 5% hydrochloric acid solution followed by separation, chloroform was added to the lower layer followed by separation, the chloroform layer was washed with 5% calcium carbonate solution and then with water, and dried. Chloroform was evaporated in vacuo, the residue was purified by silica gel column chromatography (100 grams of silica gel being used; eluting solution is ethyl acetate), and the resulting crystals were recrystallized from a mixture of ethyl acetate and ether to give 1.28 grams (yield (20%) of 2-(N-benzyl-N-methylamino)-ethyl 5-dimethoxyphosphinyl-2,6-dimethyl-4-(3-nitrophenyl)-1,4-dihydropyridine-3-carboxylate, pale yellow crystals, melting point 101—3°C.

IR spectra (KBr, $cm^{-1}$): 3380, 3250, 3100, 1705, 1650, 1535, 1350

NMR spectra ($CDCl_3$: δ): 2.20 (3H, s) 2.31 (3H, d, J=2.5Hz) 2.35 (3H, s) 2.60 ~ 2.68 (2H, m) 3.33 (3H, d, J=11Hz) 3.50 (2H, d J=2Hz) 3.54 (3H, d, J=11Hz) 4.13 ~ 4.21 (2H, m) 4.82 (1H, d, J=10Hz) 6.10 (1H, d, J=5Hz) 7.26 (5H, s) 7.33 (1H, t, J=8Hz) 7.69 (1H, d, J=8Hz) 7.95 ~ 8.01 (1H, m) 8.12 (1H, t, J=2Hz).

Elementary analysis for $C_{26}H_{32}N_3O_7P$

Calculated (%) C: 58.97 H: 6.09 N: 7.94

Determined (%) C: 58.92 H: 6.40 N: 7.94

Example 3

A mixture of 5.1 grams of diisopropyl 1-(3-nitrobenzylidene)acetonylphosphonate and 2.69 grams of 2-n-propoxyethyl 3-aminocrotonate was added to 20 ml of isopropanol and the mixture was heated to reflux for eight hours with stirring. The reaction mixture was evaporated in vacuo, to the residue was added ether to bring about crystallization, the crystals were collected by filtration, and recrystallized from a mixture of ethyl acetate and ether to afford 5.38 grams of 2-n-propoxyethyl 2,6-dimethyl-4-(3-nitrophenyl)-5-diisopropoxyphosphonyl-1,4-dihydropyridine-3-carboxylate, pale yellow crystals, melting point 125.6°C. Yield 72%.

IR spectra (KBr, $cm^{-1}$): 3290, 3230, 3110, 1695, 1645, 1535, 1350, 1240, 1210, 980.

NMR spectra ($CDCl_3$: δ): 0.90 (3H, t, J=7.5Hz) 0.95 (3H, d=6.2Hz) 1.13 (3H, d, J=5.0Hz) 1.15 (3H, d, J=8.0Hz) 1.26 (3H, d, J=6.2Hz) 1.48 ~ 1.62 (2H, m) 2.31 (3H, d, J=2.5Hz) 2.35 (3H, s) 3.34 ~ 3.42 (2H, m) 3.59 (2H, t, J=5Hz) 4.15 ~ 4.22 (2H, m) 4.29 ~ 4.50 (2H, m) 4.91 (1H, d, J=11.2Hz) 6.10 (1H, d, J=5Hz) 7.35 (1H, t, J=7.8Hz) 7.71 (1H, d, J=7.8Hz) 7.96 ~ 8.02 (1H, m) 8.12 (1H, t, J=2Hz).

Elementary analysis for $C_{25}H_{37}N_2O_8P$
Calculated (%) C: 57.25 H: 7.11 N: 5.34
Determined (%) C: 57.10 H: 7.21 N: 5.34

Example 4

A mixture of 2.44 grams of diisopropyl acetonylphosphonate, 1.15 grams of methyl 3-aminocrotonate, and 1.51 grams of m-nitrobenzaldehyde was dissolved in 10 ml of isopropanol, then 290 mg of piperidine acetate was added thereto, and the mixture was heated to reflux for sixteen hours with stirring. The reaction solution was concentrated in vacuo, to the residue was added ether, the resulting crystals were collected by filtration, washed with ether and then with water, dried, and recrystallized from ethyl acetate to afford 1.57 grams (yield 34%) of methyl 2,6-dimethyl-4-(3-nitrophenyl)-5-diisopropoxyphosphinyl-1,4-dihydropyridine-3-carboxylate, melting point 205—7°C.

IR spectra (KBr, $cm^{-1}$): 3270, 3200, 3080, 1705, 1645, 1530, 1500, 1350, 1240, 975.

NMR spectra ($CDCl_3$: δ): 0.99 (3H, d, J=6.2Hz) 1.11 (3H, d, J=6.2Hz) 1.16 (3H, d, J=6.2Hz) 1.25 (3H, d, J=6.2Hz) 2.29 (3H, d, J=2.5Hz) 2.36 (3H, s) 3.65 (3H, s) 4.20 ~ 4.40 (1H, m) 4.40 ~ 4.58 (1H, m) 4.90 (1H, d, J=10.3Hz) 5.83 (1H, d, J=5Hz) 7.36 (1H, t, J=7.8Hz) 7.67 (1H, d, J=7.8Hz) 7.97 ~ 8.03 (1H, m) 8.13 (1H, t, J=2Hz).

Elementary analysis for $C_{21}H_{29}N_2O_7P$
Calculated (%) C: 55.75 H: 6.46 N; 6.19
Determined (%) C: 55.39 H: 6.68 N: 6.21

Example 5

A mixture of 2.22 grams of diisopropyl acetonylphosphonate and 1.51 grams of m-nitrobenzaldehyde was added to 10 ml of isopropanol, 300 mg of piperidine acetate was added thereto, and the mixture was heated to reflux for three hours with stirring. To this was added 2.48 grams of 2-(N-benzyl-N-methylamino)-ethyl 3-aminocrotonate and the mixture was heated to reflux for another five hours with stirring. The reaction mixture was concentrated, the residue was dissolved in ethyl acetate, the solution was washed with 1N aqueous solution of sodium hydroxide, an aqueous solution of sodium bisulfite, and water successively, then separated after addition of 1N hydrochloric acid solution, to the lower layer was added chloroform followed by separation, the chloroform layer was washed with 5% aqueous solution of potassium carbonate and then with water, and dried. Chloroform was evaporated therefrom in vacuo, the residue was purified by silica gel column chromatography (100 grams of silica gel used; eluting solution: ethyl acetate) and the resulting crystals were recrystallised from ethyl acetate to afford 3.04 grams of 2-(N-benzyl-N-methylamino)-ethyl 2,6-dimethyl-4-(3-nitrophenyl)-5-diisopropyloxyphosphinyl-1,4-dihydropyridine-3-carboxylate, melting point 113—15°C, melting point of the hydrochloride 119°C.

IR spectra (KBr, $cm^{-1}$): 3270, 3210, 3080, 1700, 1645, 1530, 1500, 1355, 1235, 1215, 975

NMR spectra ($CDCl_3$: δ): 0.96 (3H, d, J=6.2Hz) 1.24 (3H, d, J=6Hz) 2.19 (3H, S) 2.30 (3H, d, J=2.5Hz) 2.34 (3H, s) 2.64 (2H, t, J=6Hz) 3.49 (2H, s), 4.14 ~ 4.24 (2H, m) 4.26 ~ 4.60 (2H, m) 4.91 (1H, d, J=10.1Hz) 5.79 (1H, d, J=5Hz) 7.20 ~ 7.35 (6H, m) 7.69 (1H,d, J=8Hz) 7.95 ~ 8.00 (1H, m) 8.13 (1H, t, J=1Hz).

Elementary analysis for $C_{30}H_{40}N_3O_7P$
Calculated (%) C: 61.53 H: 6.88 N: 7.18
Determined (%) C: 61.32 H: 7.06: N: 7.14

Similarly as in Examples 1 to 5 the compounds of the following Examples 6 to 62 were prepared.

Example 6

Ethyl 5-dimethoxyphosphinyl-2,6-dimethyl-4-(3-nitrophenyl)-1,4-dihydropyridine-3-carboxylate. Melting point 155—7°C.

Elementary analysis for $C_{18}H_{23}N_2O_7P$
Calculated (%) C: 52.69 H: 5.65 N: 6.83
Determined (%) C: 52.77 H: 5.85 N: 6.82

Example 7

Isopropyl 5-dimethoxyphosphinyl-2,6-dimethyl-4-(3-nitrophenyl)-1,4-dihydropyridine-3-carboxylate. Melting point 171—2°C.

Elementary analysis for $C_{19}H_{25}N_2O_7P$
Calculated (%) C: 53.77 H: 5.94 N: 6.60
Determined (%) C: 53.73 H: 6.09 N: 6.49

Example 8

Methyl 5-diethoxyphosphinyl-2,6-dimethyl-4-(3-nitrophenyl)-1,4-dihydroxypyridine-3-carboxylate. Melting point 179—81°C.

Elementary analysis for $C_{19}H_{25}N_2O_7P$
Calculated (%) C: 53.77 H: 5.94 N: 6.60
Determined (%) C: 53.56 H: 6.04 N: 6.41

Example 9

Ethyl 5-diethoxyphosphinyl-2,6-dimethyl-4-(3-nitrophenyl)-1,4-dihydropyridine-3-carboxylate. Melting point 135—7°C.

Elementary analysis for: $C_{20}H_{27}N_2O_7P$

| | | | |
|---|---|---|---|
| Calculated (%): | C: 54.79 | H: 6.21 | N: 6.39 |
| Determined (%): | C: 54.44 | H: 6.45 | N: 6.23 |

Example 10

Isopropyl 5-diethoxyphosphinyl-2,6-dimethyl-4-(3-nitrophenyl)-1,4-dihydropyridine-3-carboxylate. Melting point 151—2°C.

Elementary analysis for: $C_{21}H_{29}N_2O_7P$

| | | | |
|---|---|---|---|
| Calculated (%): | C: 55.75 | H: 6.46 | N: 6.19 |
| Determined (%): | C: 55.44 | H: 6.64 | N: 6.04 |

Example 11

2-(N-Benzyl-N-methylamino)-ethyl 5-diethoxyphosphinyl-2,6-dimethyl-4-(3-nitrophenyl)-1,4-dihydropyridine-3-carboxylate. Melting point 77—9°C.

Elementary analysis for: $C_{28}H_{36}N_3O_7P$

| | | | |
|---|---|---|---|
| Calculated (%): | C: 60.32 | H: 6.51 | N: 7.54 |
| Determined (%): | C: 60.07 | H: 6.65 | N: 7.35 |

Example 12

Ethyl 2,6-dimethyl-4-(3-nitrophenyl)-5-diisopropoxyphosphinoyl-1,4-dihydropyridine-3-carboxylate. Melting point 152—4°C.

Elementary analysis for: $C_{22}H_{31}N_2O_7P$

| | | | |
|---|---|---|---|
| Calculated (%): | C: 56.65 | H: 6.70 | N: 6.01 |
| Determined (%): | C: 56.29 | H: 6.89 | N: 5.96 |

Example 13

Isopropyl 2,6-dimethyl-4-(3-nitrophenyl)-5-diisopropoxyphosphinoyl-1,4-dihydropyridine-3-carboxylate. Melting point 134—6°C.

Elementary analysis for: $C_{23}H_{33}N_2O_7P$

| | | | |
|---|---|---|---|
| Calculated (%): | C: 57.49 | H: 6.92 | N: 5.83 |
| Determined (%): | C: 57.31 | H: 7.09 | N: 5.78 |

Example 14

2-(Dimethylamino)ethyl 2,6-dimethyl-4-(3-nitrophenyl)-5-diisopropoxyphosphinyl-1,4-dihydropyridine-3-carboxylate. Melting point 122—3°C.

Elementary analysis for: $C_{24}H_{36}N_3O_7P$

| | | | |
|---|---|---|---|
| Calculated (%): | C: 56.57 | H: 7.12 | N: 8.25 |
| Determined (%): | C: 56.35 | H: 7.13 | N: 8.05 |

Example 15

2-Benzyloxyethyl 2,6-dimethyl-4-(3-nitrophenyl)-5-diisopropoxyphosphinyl-1,4-dihydropyridine-3-carboxylate. Melting point 94—5°C.

Elementary analysis for: $C_{29}H_{37}N_2O_8P$

| | | | |
|---|---|---|---|
| Calculated (%): | C: 60.83 | H: 6.51 | N: 4.89 |
| Determined (%): | C: 60.57 | H: 6.78 | N: 4.98 |

Example 16

2-Phenoxyethyl 2,6-dimethyl-4-(3-nitrophenyl)-5-diisopropoxyphosphinyl-1,4-dihydropyridine-3-carboxylate. Melting point 144—5°C.

Elementary analysis for: $C_{28}H_{35}N_2O_8P$

| | | | |
|---|---|---|---|
| Calculated (%): | C: 60.21 | H: 6.32 | N: 5.02 |
| Determined (%): | C: 60.17 | H: 6.55 | N: 5.08 |

Example 17

Allyl 2,6-dimethyl-4-(3-nitrophenyl)-5-diisopropoxyphosphinyl-1,4-dihydropyridine-3-carboxylate. Melting point 145—6°C.

Elementary analysis for: $C_{23}H_{31}N_2O_7P$

| | | | |
|---|---|---|---|
| Calculated (%): | C: 57.74 | H: 6.53 | N: 5.85 |
| Determined (%): | C: 57.56 | H: 6.64 | N: 5.70 |

Example 18

Cyclopentyl 2,6-dimethyl-4-(3-nitrophenyl)-5-diisopropoxyphosphinyl-1,4-dihydropyridine-3-carboxylate. Melting point 153—4°C.

Elementary analysis for: $C_{25}H_{35}N_2O_7P$

Calculated (%): C: 59.28 H: 6.96 N: 5.53

Determined (%): C: 59.05 H: 7.11 N: 5.36

Example 19

N-Benzyl-4-piperidinyl 2,6-dimethyl-4-(3-nitrophenyl)-5-diisopropoxyphosphinyl-1,4-dihydropyridine-3-carboxylate. Melting point 186—7°C.

Elementary analysis for: $C_{32}H_{42}N_3O_7P$

Calculated (%): C: 62.83 H: 6.92 N: 6.87

Determined (%): C: 62.68 H: 7.16 N: 6.68

Example 20

2-(N-Benzyl-N-methylamino)-1-phenylethyl 2,6-dimethyl-4-(3-nitrophenyl)-5-diisopropoxyphosphinyl-1,4-dihydropyridine-3-carboxylate. (Isomers due to diastereomers were separated)

a) Melting point 148—9°C.

Elementary analysis for: $C_{36}H_{44}N_3O_7P$

Calculated (%): C: 65.34 H: 6.70 N: 6.35

Determined (%): C: 65.10 H: 6.69 N: 6.24

b) Another isomer, melting point 101—3°C.

Elementary analysis for: $C_{36}H_{44}N_3O_7P$

Calculated (%): C: 65.34 H: 6.70 N: 6.35

Determined (%): C: 65.01 H: 6.82 N: 6.18

Example 21

Ethyl 2,6-dimethyl-4-(3-nitrophenyl)-5-di-n-propoxyphosphinyl-1,4-dihydropyridine-3-carboxylate. Melting point 125—6°C.

Elementary analysis for: $C_{22}H_{32}N_2O_7P$

Calculated (%): C: 56.64 H: 6.70 N: 6.01

Determined (%): C: 56.47 H: 6.84 N: 6.02

Example 22

2-(N-Benzyl-N-methylamino)-ethyl 2,6-dimethyl-4-(3-nitrophenyl)-5-di-n-propoxyphosphinyl-1,4-dihydropyridine-3-carboxylate. Pale yellow oil. Picrate melts at 104—5°C.

Elementary analysis for: $C_{30}H_{40}N_3O_7P \cdot C_6H_3N_3O_7$

Calculated (%): C: 53.07 H: 5.32 N: 10.31

Determined (%): C: 53.92 H: 5.35 N: 10.27

Example 23

2-n-Propoxyethyl 2,6-dimethyl-4-(3-nitrophenyl)-5-di-n-propoxyphosphinyl-1,4-dihydropyridine-3-carboxylate. Melting point 80—1°C.

Elementary analysis for: $C_{25}H_{37}N_2O_8P$

Calculated (%): C: 57.25 H: 7.11 N: 5.34

Determined (%): C: 57.10 H: 7.19 N: 5.35

Example 24

Methyl 2,6-dimethyl-4-(3-nitrophenyl)-5-di-n-propoxyphosphinyl-1,4-dihydropyridine-3-carboxylate. Melting point 174—5°C.

Elementary analysis for: $C_{21}H_{29}N_2O_7P$

Calculated (%): C: 55.75 H: 6.46 N: 6.19

Determined (%): C: 55.51 H: 6.64 N: 6.20

Example 25

Methyl 5-diisobutoxyphosphinyl-2,6-dimethyl-4-(3-nitrophenyl)-1,4-dihydropyridine-3-carboxylate. Melting point 177—8°C.

Elementary analysis for: $C_{23}H_{33}N_2O_7P$

Calculated (%): C: 57.49 H: 6.92 N: 5.83

Determined (%): C: 57.31 H: 7.19 N: 5.90

Example 26

Ethyl 5-diisobutoxyphosphinyl-2,6-dimethyl-4-(3-nitrophenyl)-1,4-dihydropyridine-3-carboxylate. Melting point 148—9°C.

Elementary analysis for: $C_{24}H_{35}O_7P$
Calculated (%): C: 58.29 H: 7.13 N: 5.66
Determined (%): C: 57.93 H: 7.41 N: 5.67

Example 27

2-(N-Benzyl-N-methylamino)-ethyl 5-diisobutoxyphosphinyl-2,6-dimethyl-4-(3-nitrophenyl)-1,4-dihydropyridine-3-carboxylate. Pale yellow oil. Picrate melts at 104—6°C.

Elementary analysis for: $C_{32}H_{44}N_3O_7P \cdot C_6H_3N_3O_7$
Calculated (%): C: 54.16 H: 5.62 N: 9.97
Determined (%): C: 53.88 H: 5.69 N: 9.89

Example 28

2-Dimethylaminoethyl 5-diisobutoxyphosphinyl-2,6-dimethyl-4-(3-nitrophenyl)-1,4-dihydropyridine-3-carboxylate. Melting point 148.5—9.5°C.

Elementary analysis for: $C_{26}H_{40}N_3O_7P$
Calculated (%): C: 58.09 H: 7.50 N: 7.82
Determined (%): C: 58.34 H: 7.71 N: 7.96

Example 29

2-n-Propoxyethyl 5-diisobutoxyphosphinyl-2,6-dimethyl-4-(3-nitrophenyl)-1,4-dihydropyridine-3-carboxylate. Melting point 103—4°C.

Elementary analysis for: $C_{27}H_{41}N_2O_8P$
Calculated (%): C: 58.69 H: 7.48 N: 5.07
Determined (%): C: 58.70 H: 7.60 N: 5.19

Example 30

N-Benzyl-4-piperidinyl 5-diisobutoxyphosphinyl-2,6-dimethyl-4-(3-nitrophenyl)-1,4-dihydropyridine-3-carboxylate. Melting point 153—4°C.

Elementary analysis for: $C_{34}H_{46}N_3O_7P$
Calculated (%): C: 63.84 H: 7.25 N: 6.57
Determined (%): C: 63.66 H: 7.32 N: 6.53

Example 31

2-(N-Benzyl-N-methylamino)-1-phenylethyl 5-diisobutoxyphosphinyl-2,6-dimethyl-4-(3-nitrophenyl)-1,4-dihydropyridine-3-carboxylate. Pale yellow oil (as a mixture of diastereomers). Picrate melts at 122—8°C.

Elementary analysis for: $C_{38}H_{48}N_3O_7P \cdot C_6H_3N_3O_7$
Calculated (%): C: 57.51 H: 5.59 N: 9.15
Determined (%): C: 57.38 H: 5.63 N: 9.22

Example 32

Methyl 5-di-n-butoxyphosphinyl-2,6-dimethyl-4-(3-nitrophenyl)-1,4-dihydropyridine-3-carboxylate. Melting point 152—4°C.

Elementary analysis for: $C_{23}H_{33}N_2O_7P$
Calculated (%): C: 57.49 H: 6.92 N: 5.83
Determined (%): C: 57.51 H: 6.99 N: 5.85

Example 33

Ethyl 5-di-n-butoxyphosphinyl-2,6-dimethyl-4-(3-nitrophenyl)-1,4-dihydropyridine-3-carboxylate. Melting point 107—9°C.

Elementary analysis for: $C_{24}H_{35}N_2O_7P$
Calculated (%): C: 58.29 H: 7.13 N: 5.66
Determined (%): C: 58.31 H: 7.24 N: 5.66

Example 34

Ethyl 5-di-n-butoxyphosphinyl-2,6-dimethyl-4-(3-nitrophenyl)-1,4-dihydropyridine-3-carboxylate. Pale yellow oil. Picrate melts at 101—3°C.

Elementary analysis for: $C_{32}H_{44}N_3O_7P \cdot C_6H_3N_3O_7$
Calculated (%): C: 54.15 H: 5.62 N: 9.97
Determined (%): C: 54.20 H: 5.68 N: 10.09

Example 35

2-n-Propoxyethyl 5-di-n-butoxyphosphinyl-2,6-dimethyl-4-(3-nitrophenyl)-1,4-dihydropyridine-3-carboxylate. Melting point 94—6°C.

Elementary analysis for: $C_{27}H_{41}N_2O_8P$
Calculated (%): C: 58.68 H: 7.48 N: 5.07
Determined (%): C: 58.31 H: 7.69 N: 5.10

Example 36

2-(Dimethylamino)ethyl 5-di-n-butoxyphosphinyl-2,6-dimethyl-4-(3-nitrophenyl)-1,4-dihydropyridine-3-carboxylate. Melting point 101—3°C.
Elementary analysis for: $C_{26}H_{40}N_3O_7P$
Calculated (%): C: 58.09 H: 7.50 N: 7.82
Determined (%): C: 57.76 H: 7.85 N: 7.85

Example 37

Methyl 5-di-sec-butoxyphosphinyl-2,6-dimethyl-4-(3-nitrophenyl)-1,4-dihydropyridine-3-carboxylate. Melting point 164—6°C.
Elementary analysis for: $C_{23}H_{33}N_2O_7P$
Calculated (%): C: 57.49 H: 6.92 N: 5.83
Determined (%): C: 57.25 H: 7.13 N: 5.74

Example 38

Ethyl 5-di-sec-butoxyphosphinyl-2,6-dimethyl-4-(3-nitrophenyl)-1,4-dihydropyridine-3-carboxylate. Melting point 150–2°C.
Elementary analysis for: $C_{24}H_{35}N_2O_7P$
Calculated (%): C: 58.29 H: 7.13 N: 5.66
Determined (%): C: 58.13 H: 7.18 N: 5.68

Example 39

2-(N-Benzyl-N-methylamino)-ethyl 5-di-sec-butoxyphosphinyl-2,6-dimethyl-4-(3-nitrophenyl)-1,4-dihydropyridine-3-carboxylate. Melting point 114—16°C.
Elementary analysis for: $C_{32}H_{44}N_3O_7P$
Calculated (%): C: 62.63 H: 7.23 N: 6.85
Determined (%): C: 62.37 H: 7.46 N: 6.77

Example 40

2-n-Propoxyethyl 5-di-sec-butoxyphosphinyl-2,6-dimethyl-4-(3-nitrophenyl)-1,4-dihydropyridine-3-carboxylate. Melting point 104—6°C.
Elementary analysis for: $C_{27}H_{41}N_2O_8P$
Calculated (%): C: 58.68 H: 7.48 N: 5.07
Determined (%): C: 58.53 H: 7.75 N: 5.06

Example 41

Methyl 2,6-dimethyl-4-(3-nitrophenyl)-5-diisopentyloxyphosphinyl-1,4-dihydropyridine-3-carboxylate. Melting point 160—1°C.
Elementary analysis for: $C_{25}H_{37}N_2O_7P$
Calculated (%): C: 59.04 H: 7.33 N: 5.51
Determined (%): C: 58.98 H: 7.52 N: 5.54

Example 42

2-(N-Benzyl-N-methylamino)-ethyl 2,6-dimethyl-4-(3-nitrophenyl)-5-diisopentyloxyphosphinyl-1,4-dihydropyridine-3-carboxylate. Pale yellow oil. Picrate melts at 117.5—8°C.
Elementary analysis for: $C_{34}H_{48}N_3O_7P \cdot C_6H_3N_3O_7$
Calculated (%): C: 55.17 H: 5.90 N: 9.65
Determined (%): C: 54.93 H: 5.82 N: 9.53

Example 43

Methyl 5-dicyclopentyloxyphosphinyl-2,6-dimethyl-4-(3-nitrophenyl)-1,4-dihydropyridine-3-carboxylate. Melting point 202—4°C.
Elementary analysis for: $C_{25}H_{33}N_2O_7P$
Calculated (%): C: 59.52 H: 6.59 N: 5.55
Determined (%): C: 59.24 H: 6.58 N: 5.58

Example 44

2-(N-Benzyl-N-methylamino)ethyl 5-dicyclopentyloxyphosphinyl-2,6-dimethyl-4-(3-nitrophenyl)-1,4-dihydropyridine-3-carboxylate. Pale yellow oil. Picrate melts at 103—5°C.
Elementary analysis for: $C_{34}H_{44}N_3O_7P \cdot C_6H_3N_3O_7$
Calculated (%): C: 55.43 H: 5.47 N: 9.70
Determined (%): C: 55.30 H: 5.32 N: 9.52

Example 45

Methyl 2,6-dimethyl-4-(3-nitrophenyl)-5-dineopentyloxyphosphinyl-1,4-dihydropyridine-3-carboxylate. Melting point 241—2°C.

Elementary analysis for: $C_{25}H_{37}N_2O_7P$

| | | | |
|---|---|---|---|
| Calculated (%): | C: 59.04 | H: 7.33 | N: 5.51 |
| Determined (%): | C: 58.82 | H: 7.60 | N: 5.33 |

Example 46

2-(N-Benzyl-N-methylamino)-ethyl 2,6-dimethyl-4-(3-nitrophenyl)-5-dineopentyloxyphosphinyl-1,4-dihydropyridine-3-carboxylate. Pale yellow oil.

IR (film, $cm^{-1}$) 3290, 3230, 3100, 1700, 1655, 1535, 1350, 1240, 1220, 980

Example 47

Methyl 5-diallyloxyphosphinyl-2,6-dimethyl-4-(3-nitrophenyl)-1,4-dihydropyridine-3-carboxylate. Melting point 164—6°C.

Elementary analysis for: $C_{21}H_{25}N_2O_7P$

| | | | |
|---|---|---|---|
| Calculated (%): | C: 56.25 | H: 5.62 | N: 6.25 |
| Determined (%): | C: 56.12 | H: 5.67 | N: 6.09 |

Example 48

2-(N-Benzyl-N-methylamino)-ethyl 5-diallyloxyphosphinyl-2,6-dimethyl-4-(3-nitrophenyl)-1,4-dihydropyridine-3-carboxylate. Pale yellow oil. Picrate melts at 136—8°C.

Elementary analysis for: $C_{30}H_{36}N_3O_7P \cdot C_6H_3N_3O_7 \cdot \frac{1}{2}H_2O$

| | | | |
|---|---|---|---|
| Calculated (%): | C: 53.34 | H: 4.85 | N: 10.37 |
| Determined (%): | C: 53.42 | H: 4.86 | N: 10.13 |

Example 49

Methyl 5-[di-(2-methoxyethoxy)-phosphinyl]-2,6-dimethyl-4-(3-nitrophenyl)-1,4-dihydropyridine-3-carboxylate. Melting point 144—6°C.

Elementary analysis for: $C_{21}H_{29}N_2O_9P$

| | | | |
|---|---|---|---|
| Calculated (%): | C: 52.07 | H: 6.03 | N: 5.78 |
| Determined (%): | C: 51.73 | H: 6.26 | N: 5.79 |

Example 50

2-(N-Benzyl-N-methylamino)-ethyl 5-[di-(2-methoxyethoxy)-phosphinyl]-2,6-dimethyl-4-(3-nitrophenyl)-1,4-dihydropyridine-3-carboxylate. Pale yellow oil.

IR (film, $cm^{-1}$): 3240, 3200, 3085, 1700, 1530, 1350, 1240, 1220, 1095, 1035, 965, 845

Example 51

Methyl 2,6-dimethyl-4-(3-nitrophenyl)-5-diphenoxyphosphinyl-1,4-dihydropyridine-3-carboxylate. Melting point 193—5°C.

Elementary analysis for: $C_{27}H_{25}N_2O_7P$

| | | | |
|---|---|---|---|
| Calculated (%): | C: 62.31 | H: 4.84 | N: 5.38 |
| Determined (%): | C: 62.19 | H: 4.78 | N: 5.30 |

Example 52

2-(N-Benzyl-N-methylamino)-ethyl 2,6-dimethyl-4-(3-nitrophenyl)-5-diphenoxyphosphinyl-1,4-dihydropyridine-3-carboxylate. Melting point 130—5°C.

Elementary analysis for: $C_{36}H_{36}N_3O_7P$

| | | | |
|---|---|---|---|
| Calculated (%): | C: 66.15 | H: 5.55 | N: 6.43 |
| Determined (%): | C: 66.18 | H: 5.45 | N: 6.36 |

Example 53

Methyl 4-(2-chlorophenyl)-2,6-dimethyl-5-diisopropoxyphosphinyl-1,4-dihydropyridine-3-carboxylate. Melting point 174—5°C.

Elementary analysis for: $C_{21}H_{29}ClNO_5P$

| | | | |
|---|---|---|---|
| Calculated (%): | C: 57.07 | H: 6.61 | N: 3.16 |
| Determined (%): | C: 57.06 | H: 6.85 | N: 3.31 |

Example 54

2-(N-Benzyl-N-methylamino)-ethyl 4-(2-chlorophenyl)-2,6-dimethyl-5-diisopropoxyphosphinyl-1,4-dihydropyridine-3-carboxylate. Pale yellow oil.

IR (film, $cm^{-1}$): 3300, 3250, 1700, 1650, 1605, 1505, 1220, 990

Example 55

Methyl 4-(3-chlorophenyl)-2,6-dimethyl-5-diisopropoxyphosphinyl-1,4-dihydropyridine-3-carboxylate. Melting point 176—8°C.

Elementary analysis for: $C_{21}H_{29}ClNO_5P$

| | | | |
|---|---|---|---|
| Calculated (%): | C: 57.07 | H: 6.61 | N: 3.16 |
| Determined (%): | C: 57.19 | H: 6.69 | N: 3.10 |

Example 56

2-(N-Benzyl-N-methylamino)-ethyl 4-(3-chlorophenyl)-2,6-dimethyl-5-diisopropoxyphosphinyl-1,4-dihydropyridine-3-carboxylate. Pale yellow oil. Picrate melts at 130—4°C.

Elementary analysis for: $C_{30}H_{40}ClN_2O_5P \cdot C_6H_3N_3O_7$

| | | | |
|---|---|---|---|
| Calculated (%): | C: 53.76 | H: 5.38 | N: 8.70 |
| Determined (%): | C: 53.56 | H: 5.41 | N: 8.61 |

Example 57

Methyl 4-(2-cyanophenyl)-2,6-dimethyl-5-diisopropoxyphosphinyl-1,4-dihydropyridine-3-carboxylate. Melting point 162—4°C.

Elementary analysis for: $C_{22}H_{29}N_2O_5P$

| | | | |
|---|---|---|---|
| Calculated (%): | C: 61.10 | H: 6.75 | N: 6.47 |
| Determined (%): | C: 61.07 | H: 6.82 | N: 6.36 |

Example 58

2-(N-Benzyl-N-methylamino)-ethyl 4-(2-cyanophenyl)-2,6-dimethyl-5-diisopropoxyphosphinyl-1,4-dihydropyridine-3-carboxylate. Pale yellow oil.

IR (film, $cm^{-1}$): 3300, 3240, 2250, 1700, 1650, 1510, 1240, 990

Example 59

Methyl 2,6-dimethyl-5-diisopropoxyphosphinyl-4-(2-trifluoromethylphenyl)-1,4-dihydropyridine-3-carboxylate. Melting point 142—5°C.

Elementary analysis for: $C_{22}H_{29}F_3NO_5P \cdot \frac{1}{2}H_2O$

| | | | |
|---|---|---|---|
| Calculated (%): | C: 54.54 | H: 6.24 | N: 2.89 |
| Determined (%): | C: 54.69 | H: 6.59 | N: 2.96 |

Example 60

2-(N-Benzyl-N-methylamino)-ethyl 2,6-dimethyl-5-diisopropoxyphosphinyl-4-(2-trifluoromethylphenyl)-1,4-dihydropyridine-3-carboxylate. Pale yellow oil.

IR (film, $cm^{-1}$): 3290, 3240, 1700, 1645, 1500, 1230, 990

Example 61

Methyl 2,6-dimethyl-5-diisopropoxyphosphinyl-4-(3-trifluoromethylphenyl)-1,4-dihydropyridine-3-carboxylate. Melting point 191—2.5°C.

Elementary analysis for: $C_{22}H_{29}F_3NO_5P$

| | | | |
|---|---|---|---|
| Calculated (%): | C: 55.57 | H: 6.14 | N: 2.94 |
| Determined (%): | C: 55.65 | H: 6.20 | N: 2.78 |

Example 62

2-(N-Benzyl-N-methylamino)-ethyl 2,6-dimethyl-5-diisopropoxyphosphinyl-4-(3-trifluoromethylphenyl)-1,4-dihydropyridine-3-carboxylate. Pale yellow oil. Picrate melts at 114—7°C.

Elementary analysis for: $C_{31}H_{40}F_3N_2O_5P \cdot C_6H_3N_3O_7 \cdot \frac{1}{2}H_2O$

| | | | |
|---|---|---|---|
| Calculated (%): | C: 52.48 | H: 5.24 | N: 8.27 |
| Determined (%): | C: 52.46 | H: 5.39 | N: 8.25 |

Example 63

Ethyl 5-diethoxyphosphinyl-2,6-dimethyl-4-(2-nitrophenyl)-1,4-dihydropyridine-3-carboxylate. Melting point 127—30°C.

Elementary analysis for: $C_{20}H_{27}N_2O_7P$

| | | | |
|---|---|---|---|
| Calculated (%): | C: 54.79 | H: 6.21 | N: 6.39 |
| Determined (%): | C: 54.76 | H: 6.28 | N: 6.22 |

Example 64

Methyl 2,6-dimethyl-5-[di-(1-methylbutoxy)-phosphinyl]-4-(3-nitrophenyl)-1,4-dihydropyridine-3-carboxylate. Melting point 160—1°C.

Elementary analysis for: $C_{25}H_{37}N_2O_7P$

| | | | |
|---|---|---|---|
| Calculated (%): | C: 59.04 | H: 7.33 | N: 5.51 |
| Determined (%): | C: 58.87 | H: 7.48 | N: 5.31 |

Example 65

2-(N-Benzyl-N-methylamino)-ethyl 2,6-dimethyl-5-[di-(1-methylbutoxy)-phosphinyl]-4-(3-nitrophenyl)-1,4-dihydropyridine-3-carboxylate. Pale yellow oil.

IR (film, $cm^{-1}$): 3290, 3225, 3100, 1700, 1650, 1530, 1500, 1350, 1240, 1220, 975

Example 66

Methyl 5-[di-(2-ethoxyethoxy)-phosphinyl]-2,6-dimethyl-4-(3-nitrophenyl)-1,4-dihydropyridine-3-carboxylate. Melting point 129—31°C.

Elementary analysis for: $C_{23}H_{33}N_2O_9P$

Calculated (%): C: 53.90 H: 6.49 N: 5.47

Determined (%): C: 53.66 H: 6.72 N: 5.59

Example 67

2-(N-Benzyl-N-methylamino)-ethyl 5-[di-(2-ethoxyethoxy)-phosphinyl-2,6-dimethyl-4-(3-nitrophenyl)-1,4-dihydropyridine-3-carboxylate. Melting point 51—2°C.

Elementary analysis for: $C_{32}H_{44}N_3O_9P$

Calculated (%): C: 59.53 H: 6.87 N: 6.51

Determined (%): C: 59.84 H: 6.98 N: 6.52

Example 68

2-(Dimethylamino)ethyl 5-diallyloxyphosphinyl-2,6-dimethyl-4-(3-nitrophenyl)-1,4-dihydropyridine-3-carboxylate. Melting point 94—6°C.

Elementary analysis for: $C_{24}H_{32}N_3O_7P$

Calculated (%): C: 57.02 H: 6.38 N: 8.31

Determined (%): C: 56.80 H: 6.38 N: 8.35

Example 69

2-n-Propoxyethyl 5-diallyloxyphosphinyl-2,6-dimethyl-4-(3-nitrophenyl)-1,4-dihydropyridine-3-carboxylate. Melting point 91—3°C.

Elementary analysis for: $C_{25}H_{33}N_2O_8P$

Calculated (%): C: 57.69 H: 6.39 N: 5.38

Determined (%): C: 57.54 H: 6.52 N: 5.47

Example 70

Methyl 4-(2,3-dichlorophenyl)-2,6-dimethyl-5-diisopropoxyphosphinyl-1,4-dihydropyridine-3-carboxylate. Melting point 181—4°C.

Elementary analysis for: $C_{21}H_{28}Cl_2NO_5P$

Calculated (%): C: 52.95 H: 5.93 N: 2.94

Determined (%): C: 53.12 H: 6.09 N: 3.04

Example 71

2-(N-Benzyl-N-methylamino)-ethyl 4-(2,3-dichlorophenyl)-2,6-dimethyl-5-diisopropoxyphosphinyl-1,4-dihydropyridine-3-carboxylate. Melting point 109—11°C.

Elementary analysis for: $C_{30}H_{39}Cl_2N_2O_5P$

Calculated (%): C: 59.12 H: 6.49 N: 4.60

Determined (%): C: 59.15 H: 6.55 N: 4.79

Example 72

Methyl 2,6-dimethyl-4-(3-nitrophenyl)-5-[di-(tetrahydrofurfuryloxy)phosphinyl]-1,4-dihydropyridine-3-carboxylate. Melting point 170—1°C.

Elementary analysis for: $C_{25}H_{33}N_2O_9P$

Calculated (%): C: 55.97 H: 6.20 N: 5.22

Determined (%): C: 55.76 H: 6.22 N: 5.33

Example 73

2-(N-Benzyl-N-methylamino)-ethyl 2,6-dimethyl-4-(3-nitrophenyl)-5-[di-(tetrahydrofurfuryloxy)-phosphinyl]-1,4-dihydropyridine-3-carboxylate. Pale yellow oil.

IR (film, $cm^{-1}$): 3290, 3230, 3100, 1700, 1680, 1650, 1530, 1500, 1350, 1240, 1210, 1030

Similarly prepared were the additional 107 compounds given in the following table under Examples 74 to 180, inclusive. All of these compounds were identified by their elementary analyses. When the products were oil, they were identified by measuring their infra red absorption spectra. In these Examples, $R^3$ and $R^5$ are methyl groups throughout. With reference to "$R^6$, $R^7$", when only one substituent is given in the corresponding column, then R is hydrogen and $R^7$ is the said substituent as given there; when two substituents are given (i.e. "2,3—$Cl_2$"), it means that $R^6$ = 2—Cl and $R^7$ = 3—Cl.

| Example Numbers | $OR^1 = OR^2$ | $R^4$ | $R^6,R^7$ | Melting Points | Recrystallization Solvents |
|---|---|---|---|---|---|
| 74 | iso-propoxy | tetrahydrofurfuryl | 3-$NO_2$ | 170—1°C | AcOEt—$Et_2O$ |
| 75 | 2-methylallyloxy | Me | 3-$NO_2$ | 179—80 | AcOEt—$Et_2O$ |
| 76 | 2-methoxyethoxy | 2-(dimethylamino)ethyl | 3—$NO_2$ | 77—9 | $Et_2O$ |
| 77 | 2-methoxyethoxy | propoxyethyl | 3—$NO_2$ | 74—6 | $Et_2O$ |
| 78 | crotyloxy | Me | 3-$NO_2$ | 136—8 | AcOEt—$Et_2O$ |
| 79 | crotyloxy | 2-(N-benzyl-N-methylamino)-ethyl | 3-$NO_2$ | (picrate 102—4) | |
| 80 | 2-isopropoxy-ethoxy | Me | 3-$NO_2$ | 133—4 | AcOEt—$Et_2O$ |
| 81 | 2-isopropoxy-ethoxy | 2-(N-benzyl-N-methylamino)-ethyl | 3-$NO_2$ | 76—8 | $Et_2O$ |
| 82 | 2-methylallyloxy | 2-(N-benzyl-N-methylamino-ethyl | 3-$NO_2$ | (oil) | |
| 83 | allyloxy | Me | 2,3-$Cl_2$ | 181—2 | AcOEt—$Et_2O$ |
| 84 | isopropoxy | 2-(methylthio)ethyl | 3-$NO_2$ | 150.5—1 | AcOEt—$Et_2O$ |
| 85 | 1-methyl-2-propenyloxy | Me | 3-$NO_2$ | 163—5 | AcOEt |
| 86 | 1-methyl-2-propenyloxy | 2-(N-benzyl-N-methylamino)-ethyl | 3-$NO_2$ | 93—5 | $Et_2O$ |
| 87 | allyloxy | Me | 3-$CF_3$ | 122—4 | AcOEt—$Et_2O$ |
| 88 | 3-methoxy-3-methyl-butoxy | Me | 3-$NO_2$ | 130—1 | $Et_2O$ |
| 89 | 3-methoxy-3-methyl-butoxy | 2-(N-benzyl-N-methylamino)-ethyl | 3-$NO_2$ | 83—5 | $Et_2O$ |
| 90 | allyloxy | Me | 2-$CF_3$ | 105—7 | $Et_2O$-hexane |
| 91 | allyloxy | 2-(N-benzyl-N-methylamino)-ethyl | 2-$CF_3$ | (oil) | |

| Example Numbers | $OR^1 = OR^2$ | $R^4$ | $R^6,R^7$ | Melting Points | Recrystallization Solvents |
|---|---|---|---|---|---|
| 92 | 1-ethylpropoxy | Me | $3\text{-}NO_2$ | 179—80 | AcOEt |
| 93 | 1-ethylpropoxy | 2-(N-benzyl-N-methylamino)-ethyl | $3\text{-}NO_2$ | 126—7 | $Et_2O$ |
| 94 | allyloxy | 2-(N-benzyl-N-methylamino)-ethyl | $3\text{-}CF_3$ | (oil) | |
| 95 | 1,2-dimethylpropoxy | Me | $3\text{-}NO_2$ | 195—7 | $AcOEt\text{-}Et_2O$ |
| 96 | 1,2-dimethylpropoxy | 2-(N-benzyl-N-methylamino)-ethyl | $3\text{-}NO_2$ | 136—8 | $n\text{-hexane-}Et_2O$ |
| 97 | pentyloxy | 2-(N-benzyl-N-methylamino)-ethyl | $3\text{-}NO_2$ | (picrate 116—8) | |
| 98 | pentyloxy | Me | $3\text{-}NO_2$ | 134—6 | $AcOEt\text{-}Et_2O$ |
| 99 | allyloxy | 2-(N-benzyl-N-methylamino)-ethyl | $2,3\text{-}Cl_2$ | (oil) | |
| 100 | isopropoxy | 3-pyridylmethyl | $3\text{-}NO_2$ | 175—6 | $AcOEt\text{-}Et_2O$ |
| 101 | isopropoxy | 3-methoxy-3-methylbutyl | $3\text{-}NO_2$ | 130—1 | $Et_2$ |
| 102 | isopropoxy | 2-methoxy-1-(methoxymethyl)-ethyl | $3\text{-}NO_2$ | 125—5.5 | $Et_2O$ |
| 103 | isopropoxy | 2-(4-benzyl-1-piperazinyl)-ethyl | $3\text{-}NO_2$ | 154—5 | $Et_2O$ |
| 104 | 2-methoxyethoxy | Me | $3\text{-}CF_3$ | 111—3 | AcOEt |
| 105 | 2-methoxyethoxy | Me | $2\text{-}CF_3$ | 105—7 | $AcOEt\text{-}Et_2O$ |
| 106 | 2-methoxyethoxy | Me | $2,3\text{-}Cl_2$ | 133—5 | $AcOEt\text{-}Et_2O$ |

| Example Numbers | $OR^1 = OR^2$ | $R^4$ | $R^6,R^7$ | Melting Points | Recrystallization Solvents |
|---|---|---|---|---|---|
| 107 | 2-methoxyethoxy | 2-(N-benzyl-N-methylamino)-ethyl | $3-CF_3$ | (oil) | |
| 108 | 2-methylallyloxy | Me | $3-CF_3$ | 145—7 | $AcOEt-Et_2O$ |
| 109 | 1-methylpentyloxy | Me | $3-NO_2$ | 137—9 | $AcOEt-Et_2O$ |
| 110 | 1-methylpentyloxy | 2-(N-benzyl-N-methylamino)-ethyl | $3-NO_2$ | 61—3 | $n-hexane-Et_2O$ |
| 111 | 1-ethylbutoxy | Me | $3-No_2$ | 153—5 | $AcOEt-Et_2O$ |
| 112 | 1-ethylbutoxy | 2-(N-benzyl-N-methylamino)-ethyl | $3-NO_2$ | 97—9 | $n-hexane-Et_2O$ |
| 113 | 1-methylbutoxy | Me | $2,3-Cl_2$ | 152—4 | $n-hexane-Et_2O$ |
| 114 | 1-methylbutoxy | Me | $3-CF_3$ | 116—7 | $n-hexane-Et_2O$ |
| 115 | isopropoxy | benzyl | $3-NO_2$ | 150—1 | $AcOEt-Et_2O$ |
| 116 | hexyloxy | Me | $3-NO_2$ | 121—3 | $n-hexane-Et_2O$ |
| 117 | hexyloxy | 2-(N-benzyl-N-methylamino)-ethyl | $3-NO_2$ | (picrate 117—9) | |
| 118 | heptyloxy | Me | $3-NO_2$ | 124—6 | AcOEt-hexane |
| 119 | heptyloxy | 2-(N-benzyl-N-methylamino)-ethyl | $3-NO_2$ | (picrate 82—4) | |
| 120 | 2-methylallyloxy | 2-(N-benzyl-N-methylamino)-ethyl | $3-CF_3$ | (oil) | |
| 121 | 2-methylallyloxy | 2-(N-benzyl-N-methylamino)-ethyl | $2,3-Cl_2$ | (oil) | |

| Example Numbers | $OR^1 = OR^2$ | $R^4$ | $R^6,R^7$ | Melting Points | Recrystallization Solvents |
|---|---|---|---|---|---|
| 122 | 1-methylbutoxy | 2-(N-benzyl-N-methylamino)-ethyl | 3-$CF_3$ | (oil) | |
| 123 | 1-methylbutoxy | 2-(N-benzyl-N-methylamino)-ethyl | 2,3-$Cl_2$ | (oil) | |
| 124 | 2-methoxyethoxy | 2-(N-benzyl-N-methylamino)-ethyl | 2-$CF_3$ | (oil) | |
| 125 | 2-methoxyethoxy | 2-(N-benzyl-N-methylamino)-ethyl | 2,3-$Cl_2$ | (oil) | |
| 126 | isopropoxy | 2-(N-benzyl-N-methylamino)-1-(N-benzyl-N-methylamino-methyl)-ethyl | 3-$NO_2$ | (oil) | |
| 127 | 1,3-dimethylbutoxy | Me | 3-$NO_2$ | 149—9.5 | n-hexane-AcOEt |
| 128 | 1,1,2-trimethylpropoxy | Me | 3-$NO_2$ | 221—3 | n-hexane-AcOEt |
| 129 | 1,1,2-trimethylpropoxy | 2-(N-benzyl-N-methylamino)-ethyl | 3-$NO_2$ | 165—7 | n-hexane-AcOEt |
| 130 | 3-methyl-3-butenyloxy | Me | 3-$NO_2$ | 147—9 | n-hexane-AcOEt |
| 131 | 3-methyl-3-butenyloxy | 2-(N-benzyl-N-methylamino)-ethyl | 3-$NO_2$ | (picrate 110—2) | |
| 132 | tetrahydrofurfuryloxy | 2-methoxy-1-methoxymethyl-ethyl | 3-$NO_2$ | (oil) | |

| Example Numbers | $OR^1 = OR^2$ | $R^4$ | $R^6,R^7$ | Melting Points | Recrystallization Solvents |
|---|---|---|---|---|---|
| 133 | tetrahydrofurfuryloxy | 3-pyridinyl-methyl | $3\text{-}NO_2$ | 124—6 | $AcOEt\text{-}Et_2O$ |
| 134 | 2-isopropoxyethoxy | 2-(N-benzyl-N-methylamino)-ethyl | $2,3\text{-}Cl_2$ | (oil) | |
| 135 | 2-isopropoxyethoxy | 2-(N-benzyl-N-methylamino)-ethyl | $3\text{-}CF_3$ | (oil) | |
| 136 | 2-methylallyloxy | Me | $2\text{-}CF_3$ | 141—2 | $AcOEt\text{-}Et_2O$ |
| 137 | 1,3-dimethylbutoxy | 2-(N-benzyl-N-methylamino)-ethyl | $3\text{-}NO_2$ | (oil) | |
| 138 | 1,3-dimethylbutoxy | 2-(N-benzyl-N-methylamino)-ethyl | $3\text{-}NO_2$ | (oil) | |
| 139 | 1-ethylallyloxy | Me | $3\text{-}NO_2$ | 179—80 | $AcOEt\text{-}Et_2O$ |
| 140 | 1-ethylallyloxy | 2-(N-benzyl-N-methylamino)-ethyl | $3\text{-}NO_2$ | 113—4 | n-hexane-AcOEt |
| 141 | 2-methylallyloxy | 2-(N-benzyl-N-methylamino)-ethyl | $2\text{-}CF_3$ | (oil) | |
| 142 | 3-methoxy-3-methylbutoxy | 2-(N-benzyl-N-methylamino)-ethyl | $3\text{-}CF_3$ | (oil) | |
| 143 | 3-methoxy-3-methylbutoxy | 2-(N-benzyl-N-methylamino)-ethyl | $2\text{-}CF_3$ | (oil) | |
| 144 | 2-isopropoxyethoxy | 2-(N-benzyl-N-methylamino)-ethyl | $2\text{-}CF_3$ | (oil) | |

| Example Numbers | $OR^1 = OR^2$ | $R^4$ | $R^6,R^7$ | Melting Points | Recrystallization Solvents |
|---|---|---|---|---|---|
| 145 | cyclopropylmethoxy | 2-(N-benzyl-N-methylamino)-ethyl | 3-$NO_2$ | (picrate 101—3) | |
| 146 | benzyloxy | 2-(N-benzyl-N-methylamino)-ethyl | 3-$NO_2$ | (picrate 119—21) | |
| 147 | cyclopropylmethoxy | Me | 3-$NO_2$ | 186—8 | AcOEt-$Et_2O$ |
| 148 | benzyloxy | Me | 3-$NO_2$ | 172—4 | AcOEt-$Et_2O$ |
| 149 | isopropoxy | 2-pyridinyl-methyl | 3-$NO_2$ | 148—9 | AcOEt-$Et_2O$ |
| 150 | 3-butenyloxy | Me | 3-$NO_2$ | 148—50 | AcOEt-$Et_2O$ |
| 151 | 3-butenyloxy | 2-(N-benzyl-N-methyl-amino)-ethyl | 3-$NO_2$ | (picrate 102—4) | |
| 152 | 2-isopropoxy ethoxy | Me | 3-$CF_3$ | 89—91 | n-hexane-$Et_2O$ |
| 153 | 2-isopropoxy ethoxy | Me | 2-$CF_3$ | (oil) | |
| 154 | 2-isopropoxy ethoxy | Me | 2,3-$Cl_2$ | 113—5 | iso-$Pr_2O$ |
| 155 | 3-methoxy-3-methylbutoxy | Me | 3-$CF_3$ | 128—9 | n-hexane-$Et_2O$ |
| 156 | 3-methoxy-3-methylbutoxy | Me | 2-$CF_3$ | 65—8 | n-hexane-$Et_2O$ |
| 157 | tetrahydro-furfuryloxy | 2-(N-benzyl-N-methyl-amino)-ethyl | 3-$CF_3$ | (oil) | |
| 158 | tetrahydro-furfuryloxy | Me | 3-$CF_3$ | 123—4 | iso-$Pr_2O$ |

| Example Numbers | $OR^1 = OR^2$ | $R^4$ | $R^6,R^7$ | Melting Points | Recrystallization Solvents |
|---|---|---|---|---|---|
| 159 | 3-methylbutenyloxy | 2-(N-benzyl-N-methyl-amino)-ethyl | 2-$CF_3$ | (oil) | |
| 160 | 2-methylallyloxy | Me | 2,3-$Cl_2$ | 133—5 | iso-$Pr_2O$ |
| 161 | 1-methylbutoxy | Me | 2-$CF_3$ | 119—20 | n-hexane |
| 162 | allyloxy | 2-(N-benzyl-N-methyl-amino)-ethyl | 2-$NO_2$ | (oil) | |
| 163 | 3-butynyloxy | Me | 3-$NO_2$ | 140—4 | n-hexane-AcOEt |
| 164 | allyloxy | Me | 2-$NO_2$ | 132—3 | n-hexane-AcOEt |
| 165 | 2-propynyloxy | Me | 3-$NO_2$ | 185—6 | $CH_3CN$ |
| 166 | 2-propynyloxy | 2-(N-benzyl-N-methyl-amino)-ethyl | 3-$NO_2$ | 102—3 | AcOEt-$Et_2O$ |
| 167 | 1-methylhexyloxy | Me | 3-$NO_2$ | 123.5—4.5 | n-hexane-AcOEt |
| 168 | 1-methylbutoxy | 2-(N-benzyl-N-methyl-amino)-ethyl | 2-$CF_3$ | (oil) | |
| 169 | 2-methoxyethoxy | Me | 2-$NO_2$ | 103—5 | n-hexane-AcOEt |
| 170 | 3-butynyloxy | 2-(N-benzyl-N-methyl-amino)-ethyl | 3-$NO_2$ | 92—4 | $Et_2O$ |
| 171 | 2-methoxyethoxy | 2-(N-benzyl-N-methyl-amino)-ethyl | 2-$NO_2$ | (oil) | |
| 172 | isopropoxy | Me | 2-$NO_2$ | 164—6 | n-hexane-AcOEt |
| 173 | methallyloxy | Me | 2-$NO_2$ | 149—52 | |
| 174 | isopropoxy | 2-(N-benzyl-N-methyl-amino)-ethyl | 2-$NO_2$ | (oil) | |
| 175 | 1-methylhexyloxy | 2-(N-benzyl-N-methyl-amino)-ethyl | 3-$NO_2$ | (oil) | |

| Example Numbers | $OR^1 = OR^2$ | $R^4$ | $R^6,R^7$ | Melting Points | Recrystallization Solvents |
|---|---|---|---|---|---|
| 176 | 2-propynyloxy | Me | 2-$NO_2$ | 144.5—6 | |
| 177 | 2-propynyloxy | 2-(N-benzyl-N-methyl-amino)-ethyl | 2-$NO_2$ | (oil) | |
| 178 | allyloxy | Me | 2-$OCHF_2$ | 129—30 | iso-$Pr_2O$ |
| 179 | allyloxy | 2-(N-benzyl-N-methyl-amino)-ethyl | 2-$OCHF_2$ | (oil) | |
| 180 | allyloxy | cyclopropylmethyl | 3-$NO_2$ | 135.5 | |

**Claims**

1. 1,4-Dihydropyridine-3-carboxylates represented by the following general formula (I) and salts thereof:

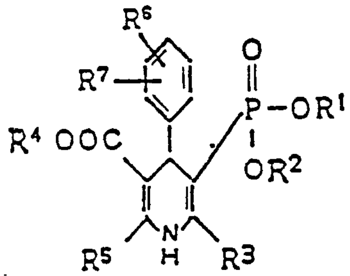

( I )

in which

$R^1$ and $R^2$ are same or different groups standing for: hydrogen; a hydrocarbyl radical of one to ten carbon atoms containing zero to four unsaturated bonds, and optionally substituted by halogen atom(s) and /or alkoxy group(s); or a tetrahydrofurfuryl group optionally substituted by alkyl group(s);

$R^3$ is lower alkyl group;

$R^4$ is: hydrogen; a hydrocarbyl radical of one to ten carbon atoms containing zero to four unsaturated bonds and optionally substituted by alkoxy, aryloxy, aralkyloxy, amino or alkyl-substituted amino group(s); a group represented by

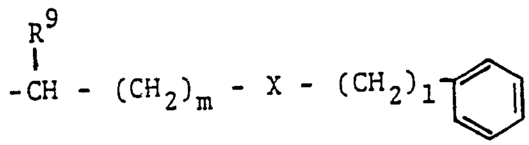

(in which X is $\overset{R^8}{\overset{|}{N}}$ or O; wherein $R^8$ is lower alkyl or lower alkenyl group; $R^9$ is hydrogen, an alkyl group or a phenyl group optionally substituted by alkyl group(s); l is an integer of 0 to 2; m is an integer of 1 to 4); or a group represented by

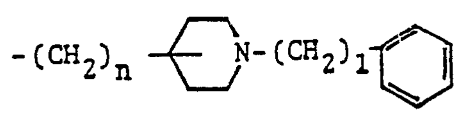

(in which l is the same as already defined; n is an integer of 0 to 2);

$R^5$ is lower alkyl group;

$R^6$ and $R^7$ are same or different groups standing for hydrogen, nitro, cyano, trifluoromethyl, halogen, azido, alkoxycarbonyl, aminocarbonyl, sulfamoyl or alkylsulfonyl or difluoromethoxy, provided that one of $R^6$ and $R^7$ is not hydrogen, and including the following compounds tetrahydrofurfuryl 2,6 - dimethyl - 4 - (3 - nitrophenyl) - 5 - bis(isopropoxy)phosphinyl - 1,4 - dihydropyridine - 3 - carboxylate; 2 - (methylthio)ethyl 2,6 - dimethyl - 4 - (3 - nitrophenyl) - 5 - bis(isopropoxy)phosphinyl - 1,4 - dihydropyridine - 3 - carboxylate; 3 - pyridylmethyl 2,6 - dimethyl - 4 - (3 - nitrophenyl) - 5 - bis(isopropoxy)phosphinyl - 1,4 - dihydropyridine - 3 - carboxylate; 2 - (4 - benzyl - 1 - piperazinyl) - ethyl 2,6 - dimethyl - 4 - (3 - nitrophenyl) - 5 - bis(isopropoxy)phosphinyl - 1,4 - dihydropyridine - 3 - carboxylate; 2 - (N - benzyl - N - methylamino) - 1 - (N - benzyl - N - methylaminomethyl) - ethyl 2,6 - dimethyl - 4 - (3 - nitrophenyl) - 5 - bis(isopropoxy)phosphinyl - 1,4 - dihydropyridine - 3 - carboxylate; 3 - pyridylmethyl 2,6 - dimethyl - 4 - (3 - nitrophenyl) - 5 - bis(tetrahydrofurfuryloxy)phosphinyl - 1,4 - dihydropyridine - 3 - carboxylate; and 2 - pyridylmethyl 2,6 - dimethyl - 4 - (3 - nitrophenyl) - 5 - bis-(isopropoxy)phosphinyl - 1,4 - dihydropyridine - 3 - carboxylate.

2. A compound according to Claim 1 in which $R^1$ and $R^2$ are phenyl groups.

3. A method for preparing 1,4-dihydropyridine-3-carboxylates as claimed in Claim 1, characterized by (a) condensing in an organic solvent a benzaldehyde derivative of a formula (IV)

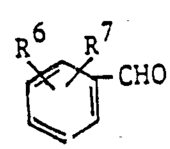

(IV)

wherein $R^6$ and $R^7$ are as defined in claim 1, with a compound of a formula (II)

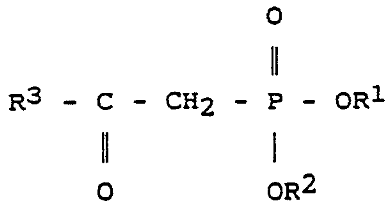

(II)

wherein $R^1$, $R^2$ and $R^3$ are as defined in claim 1, and reacting the resulting compound of a formula (V)

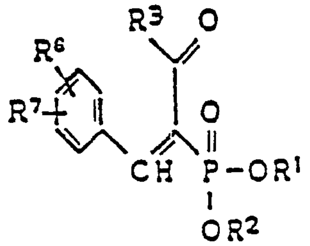

( V )

wherein $R^1$, $R^2$, $R^3$, $R^6$ and $R^7$ are as defined above, in an organic solvent with a compound of a formula (III)

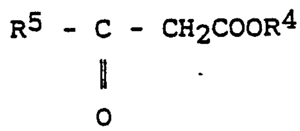

(III),

wherein $R^4$ and $R^5$ are as defined in claim 1, and ammonia or an ammonium salt, or with an enaminoester of the formula (III—N)

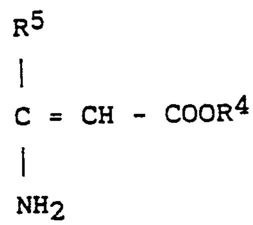

(III-N) ;

wherein $R^4$ and $R^5$ are as defined above;

or (b) reacting compounds of the formulae (IV), (II) and (III—N) in an organic solvent;

or (c) by reacting compounds of the formulae (IV), (II) and (III) and ammonia or an ammonium salt in an organic solvent.

4. A method of preparing 1,4-dihydropyridine-3-carboxylates as claimed in Claim 1, characterized by (a) condensing a benzaldehyde derivative of a formula (IV)

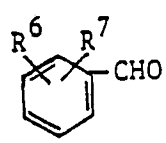

(IV)

wherein $R^6$ and $R^7$ are as defined in claim 1, in an organic solvent with a compound of the formula (III)

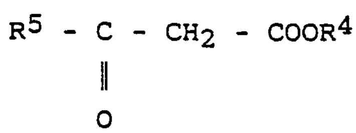

(III),

wherein $R^4$ and $R^5$ are as defined in claim 1, and reacting the resulting compound (VI)

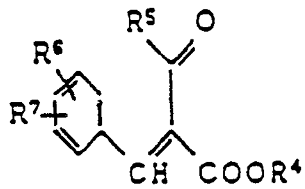

( VI )

wherein $R^4$, $R^5$, $R^6$ and $R^7$ are as defined in claim 1, in an organic solvent with a compound of a formula (II)

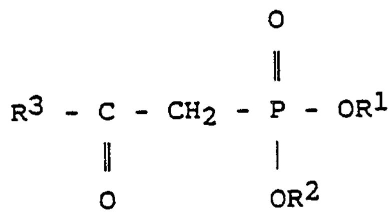

(II)

wherein $R^1$, $R^2$ and $R^3$ are as defined in claim 1, and ammonia or an ammonium salt, or with an enaminoester of the formula (II—N)

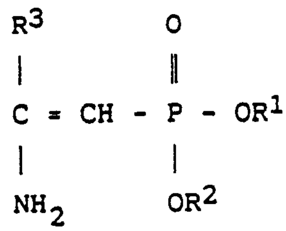

(II-N),

wherein $R^1$, $R^2$ and $R^3$ are as defined above;

or (b) reacting compounds of the formulae (IV), (III) and (II—N) in an organic solvent;

5. Pharmaceutical compositions containing 1,4-dihydropyridine-3-carboxylates as claimed in Claim 1; or salts thereof as the active ingredient for use in the treatment of diseases of the circulation.

6. Pharmaceutical compositions according to Claim 5 for use in the treatment of angina pectoris.

7. Pharmaceutical compositions according to Claim 5 for use in the treatment of hypertension.

**Patentansprüche**

1. Derivate der 1,4-Dihydropyridin-3-carbonsäure der allgemeinen Formel (I) und deren Salze:

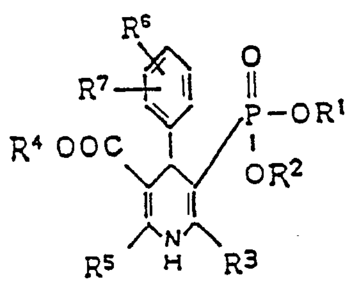

( I )

wobei

$R^1$ und $R^2$, die gleich oder verschieden sein können, Wasserstoff, einen Kohlenwasserstoffrest von 1 bis 10 Kohlenstoffatomen, der Null bis vier ungesättigte Bindungen enthält und der gegebenenfalls durch ein oder mehrere Halogenatome und/oder ein oder mehrere Alkoxygruppen substituiert sein kann; oder einen Tetrahydrofurylrest, der gegebenenfalls durch eine oder mehrere Alkylgruppen substituiert sein kann, bedeuten;

$R^3$ ist eine niedere Alkylgruppe;

$R^4$ bedeutet Wasserstoff, einen Kohlenwasserstoffrest mit 1 bis 10 Kohlenstoffatomen, der Null bis vier ungesättigte Bindungen enthält und gegebenenfalls durch eine oder mehrere alkoxy-, aryloxy-, aralkyloxy-, amino- oder alkylsubstituierte Aminogruppen substituiert ist;

eine Gruppe der Formel

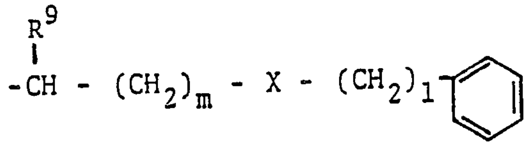

$R^8$

(in der X = N oder O; bedeutet, wobei $R^8$ einen niederen Alkylrest oder einen niederen Alkenylrest darstellt, und $R^9$ Wasserstoff, einen Alkylrest oder einen Phenylrest, der gegebenenfalls durch eine oder mehrere Alkylgruppen sustituiert sein kann, bedeuten, 1 ist eine ganze Zahl von 0 bis 2 und m ist eine ganze Zahl von 1 bis 4);
oder eine Gruppe der Formel

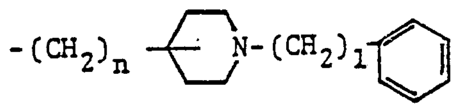

(in der 1 die gleichen Bedeutung wie oben bestizt und n eine ganze Zahl von 0 bis 2 bedeutet);

$R^5$ ist eine niedere Alkylgruppe;

$R^6$ und $R^7$, die gleich oder verschieden sein können, bedeuten Wasserstoff, Halogen, eine Nitro-, Cyano-, Trifluormethyl-, Azido-, Alkoxycarbonyl-, Aminocarbonyl-, Sulfamoyl-, Alkylsulfonyl- oder Difluormethoxygruppe, wobei jedoch einer des Reste $R^6$ oder $R^7$ nicht Wasserstoff sein darf, wobei die folgenden Verbindungen unter die allgemeine Formel fallen: 2,6 - Dimethyl - 4 - (3 - nitrophenyl) - 5 - bis(isopropoxy)phosphinyl - 1,4 - dihydropyridin - 3 - carbonsäure-tetrahydrofurylester; 2,6 - Dimethyl - 4 - (3 - nitrophenyl) - 5 - bis(isopropoxy)phosphinyl - 1,4 - dihydropyridine - 3 - carbonsäure-2-(methylthio)ethylester; 2,6 - Dimethyl - 4 - (3 - nitrophenyl) - 5 - bis(isopropoxy)phosphinyl - 1,4 - dihydropyridin - 3 - carbonsäure-3 - pyridylmethyl)ester; 2,6 - Dimethyl - 4 - (3 - nitrophenyl) - 5 - bis-(isopropoxy)phosphinyl - 1,4 - dihydropyridin - 3 - carbonsäure(2 - (4 - benzyl - 1 - piperazinyl) - ethyl)-ester; 2,6 - Dimethyl - 4 - (3 - nitrophenyl) - 5 - bis(isopropoxy)phosphinyl - 1,4 - dihydropyridin - 3 - carbonsäure (2 - (N - benzyl - N - methylamino) - 1 - (N - benzyl - N - methylaminomethyl) - ethyl) - ester; 2,6 - Dimethyl - 4 - (3 - nitrophenyl) - 5 - bis(tetrahydrofuryloxy)phosphinyl - 1,4 - dihydropyridin - 3 - carbonsäure (3 - pyridylmethyl) - ester; und 2,6 Dimethyl - 4 - (3 - nitrophenyl) - 5 - bis(isopropoxy)phosphinyl-1,4 - dihydropyridine - 3 - carbonsäure - (2-pyridylmethyl) - ester.

2. Eine Verbindung gemäß Anspruch 1, in welcher $R^1$ und $R^2$ Phenylgruppen darstellen.

3. Verfahren zur Herstellung von Derivaten der 1,4-Dihydropyridin-3-carbonsäure gemäß Anspruch 1, dadurch gekennzeichnet, daß (a) ein Benzaldehydderivat der Formel (IV)

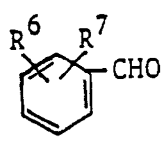

(IV)

in der $R^6$ und $R^7$ die im Anspruch 1 angeführten Bedeutungen besitzen, mit einer Verbindung der Formel (II)

$$R^3-\underset{\underset{O}{\|}}{C}-CH_2-\overset{\overset{O}{\|}}{\underset{\underset{OR^2}{|}}{P}}-OR^1 \qquad (II)$$

in der $R^1$, $R^2$ und $R^3$ die im Anspruch 1 angeführten Bedeutungen besitzen, in einem organischen Lösungsmittel kondensiert wird und die erhaltene Verbindung der Formel (V)

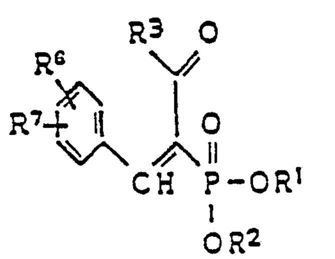

( V )

in der $R^1$, $R^2$, $R^3$, $R^6$ und $R^7$ die oben angeführten Bedeutungen besitzen, in einem organischen Lösungsmittel mit einer Verbindung der Formel (III)

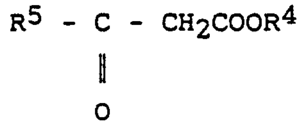

(III),

wobei $R^4$ und $R^5$ die in Anspruch 1 angeführten Bedeutungen aufweisen, und mit Ammoniak oder einem Ammonium-salz oder mit einem Enaminester der Formel (III—N)

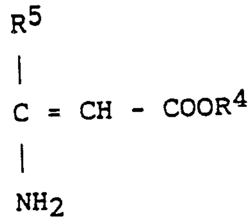

(III-N) ;

in der $R^4$ und $R^5$ die oben angeführten Bedeutungen aufweisen, in einem organischen Lösungsmittel umgesetzt wird; oder daß b) Verbindung der Formeln (IV), (II) und (III—N) einem organischen Lösungsmittel umgesetzt werden; oder daß c) Verbindungen der Formeln (IV), (II) und (III) und Ammoniak oder ein Ammoniumsalz in einem organischen Lösungsmittel umgesetzt werden.

4. Verfahren zur Herstellung von Derivaten der 1,4-Dihydropyridin-3-carbonsäure gemäß Anspruch 1, dadurch gekennzeichnet, daß (a) ein Benzaldehydderivat der Formel (IV)

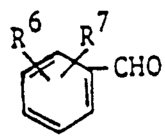

(IV)

in der $R^6$ und $R^7$ die im Anspruch 1 angeführten Bedeutungen aufweisen, mit einer Verbindung der Formel (III)

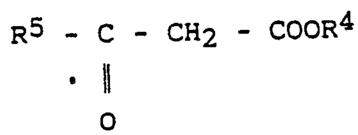

(III),

in der $R^4$ und $R^5$ die im Anspruch 1 angeführten Bedeutungen aufweisen, in einem organischen Lösungsmittel umgesetzt wird und die erhaltene Verbindung der Formel (VI)

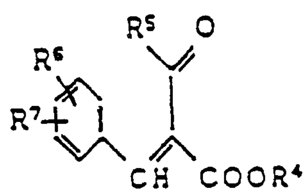

( VI )

in der $R^4$, $R^5$, $R^6$ und $R^7$ die im Anspruch 1 angeführten Bedeutungen aufweisen, in einem organischen Lösungsmittel mit einer Verbindung der Formel (II)

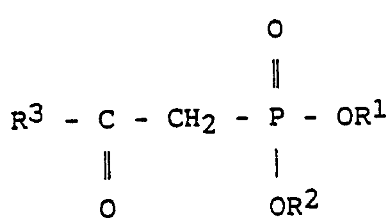

(II)

in der $R^1$, $R^2$ und $R^3$ die im Anspruch 1 angeführten Bedeutungen aufweisen, und mit Ammoniak oder einem Ammoniumsalz oder mit einem Enaminester der Formel (II—N)

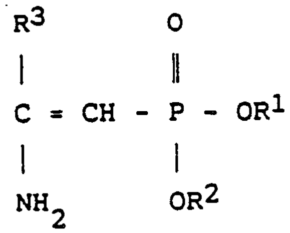

(II-N),

in der $R^1$, $R^2$ und $R^3$ die oben angeführte Bedeutung besitzen; oder daß b) Verbindung der Formeln (IV), (III) und (III—N) in einem organischen Lösungsmittel umgesetzt werden.

5. Pharmazeutische Zusammensetzungen zur Behandlung von Erkrankungen des Kreislaufs, dadurch gekennzeichnet, daß sie als Wirkstoff Derivate der 1,4-Dihydropyridin-3-carbonsäure gemäß Anspruch 1 oder eines ihrer Salze enthalten.

6. Pharmazeutische Zusammensetzungen gemäß Anspruch 5 zur Behandlung der Angina pectoris.

7. Pharmazeutische Zusammensetzungen gemaß Anspruch 5 zur Behandlung von Bluthochdruck.

**Revendications**

1. 1,4-dihydroxypyridine-3-carboxylates représentés par la formule générale (I) ci-après, et leurs sels:

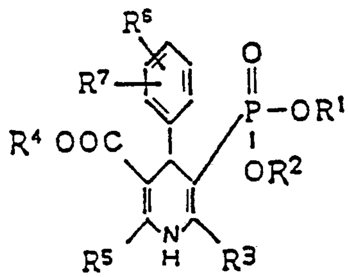

( I )

où:

$R^1$ et $R^2$ sont des groupes identiques ou différents, et représentent chacun: un hydrogène; un radical hydrocarbyle ayant de 1 à 10 atomes de carbone, contenant de zéro à quatre liaisons insaturées, et facultativement substitué par un ou plusieurs atomes d'halogène et/ou un ou plusieurs groupes alcoxy; ou un groupe tétrahydrofurfuryle facultativement substitué par un ou plusieurs groupes alkyle;

$R^3$ est un groupe alkyle inférieur;

$R^4$ est: un hydrogène; un radical hydrocarbonyle ayant de 1 à 10 atomes de carbone, contenant de zéro à quatre liaisons insaturées et facultativement substitué par un ou plusieurs groupes alcoxy, aryloxy, aralkloxy, amino ou amino alkyl-substitué; un groupe représenté par

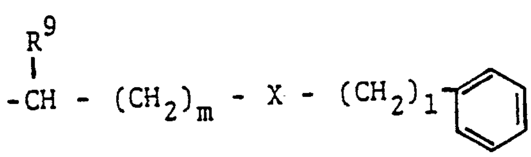

(dans laquelle X est 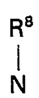 ou O; où $R^8$ est un groupe alkyle inférieur ou alcényle inférieur; $R^9$ est un hydrogène, un groupe alkyle ou un groupe phényle facultativement substitué par un ou plusieurs groupes alkyle; 1 est un entier ayant pour valeur 0 à 2; m est un entier ayant pour valeur 1 à 4); ou,

un groupe représenté par

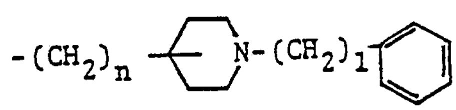

(où 1 est comme indiqué ci-dessus; n est un entier de 0 à 2);

$R^5$ est un groupe alkyle inférieur;

$R^6$ et $R^7$ sont des groupes identiques ou différents et représentent chacun un hydrogène ou un groupe nitro, cyano, trifluorométhyle, halogéno, azido, alcoxycarbonyle, aminocarbonyle, sulfamoyle, alkylsulfonyle ou difluorométhoxy, à la condition que l'un des radicaux $R^6$ ou $R^7$ ne soit pas un hydrogène,

et comprenant les composés suivants: 2,6 - diméthyl - 4 - (3 - nitrophényl) - 5 - bis(isopropoxy)-phosphinyl - 1,4 - dihydropyridine - 3 - carboxylate de tétrahydrofurfuryl; 2,6 - diméthyl - 4 - (3 - nitrophényl) - 5 - bis(isopropoxy)phosphinyl - 1,4 - dihydropyridine - 3 - carboxylate de 2-(méthylthio)éthyle; 2,6 - diméthyl - 4 - (3 - nitrophényl) - 5 - bis(isopropoxy)phosphinyl - 1,4 - dihydropyridine - 3 - carboxylate de 3 - pyridylméthyle; 2,6 - diméthyl - 4 - (3 - nitrophényl) - 5 - bis(isopropoxy)-phosphinyl - 1,4 - dihydropyridine - 3 - carboxylate de 2 - (4 - benzyl - 1 - pipérazinyl) - éthyle; 2,6 - diméthyl - 4 - (3 - nitrophényl) - 5 - bis(isopropoxy)phosphinyl - 1,4 - dihydropyridine - 3 - carboxylate de 2 - (N - benzyl - N - méthylamino) - 1 - (N - benzyl - N - méthylaminométhyl) - éthyle; 2,6 - diméthyl - 4 - (3 - nitrophényl) - 5 - bis(tétrahydrofurfuryloxy)phosphinyl - 1,4 - dihydropyridine - 3 - carboxylate de 3 - pyridylméthyle et 2,6 diméthyl - 4 - (3 - nitrophényl) - 5 - bis(isopropoxy)phosphinyl-1,4 - dihydropyridine - 3 - carboxylate de 2-pyridylméthyle.

2. Composé selon la revendication 1, dans lequel chacun des radicaux $R^1$ et $R^2$ est un groupe phényle.

3. Procédé pour la préparation de 1,4-dihydropyridine-3-carboxylates selon la revendication 1, caractérisé en ce qu'il consiste (a) à condenser dans un solvant organiques un dérivé du benzaldéhyde de formule (IV)

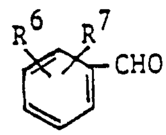

(IV)

dans laquelle $R^6$ et $R^7$ sont comme définis dans la revendication 1, avec un composé de formule (II)

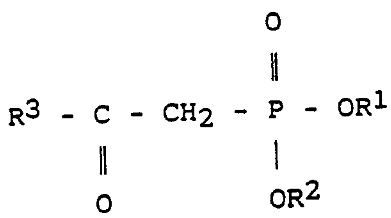

(I)

dans laquelle $R^1$, $R^2$ et $R^3$ sont comme définis dans la revendication 1, et à faire réagir le composé obtenu, de formule (V)

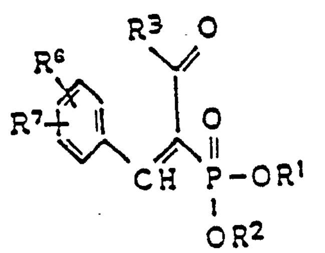

( V )

dans laquelle $R^1$, $R^2$, $R^3$, $R^6$ et $R^7$ sont comme définis ci-dessus, dans un solvant organique, avec un composé de formule (III)

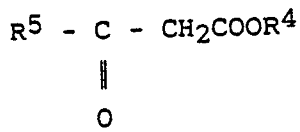

(III),

dans laquelle $R^4$ et $R^5$ sont comme définis dans la revendication 1, et de l'ammoniac ou un sel d'ammonium, ou avec un énaminoester de formule (III—N)

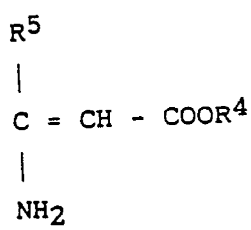

(III-N) ;

dans laquelle $R^4$ et $R^5$ sont comme définis ci-dessus;

ou (b) à faire réagir des composés de formules (IV), (II) et (III—N) dans un solvant organique;

ou (c) à faire réagir des composés de formules (IV), (II) et (III), et de l'ammoniac et un sel d'ammonium, dans un solvant organique.

4. Procédé pour la préparation de 1,4-dihydropyridine-3-carboxylates selon la revendication 1, caractérisé en ce qu'il consiste (a) à condenser un dérivé de benzaldéhyde de formule (IV)

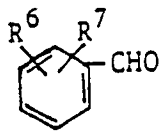

(IV)

dans laquelle $R^6$ et $R^7$ sont comme définis dans la revendication 1, dans un solvant organique avec un composé de formule (III)

```
R5 - C - CH2 - COOR4        (III),
     ‖
     O
```

dans laquelle $R^4$ et $R^5$ sont comme définis dans la revendication 1, et à faire réagir le composé obtenu (VI)

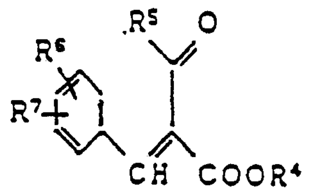

(VI)

où $R^4$, $R^5$, $R^6$ et $R^7$ sont comme définis dans la revendication 1, dans un solvant organique, avec un composé de formule (II)

```
                    O
                    ‖
R3 - C - CH2 - P - OR1        (II)
     ‖              |
     O             OR2
```

dans laquelle $R^1$, $R^2$ et $R^3$ sont comme définis dans la revendication 1, et de l'ammoniac ou un sel d'ammonium ou avec un énaminoester de formule (II—N)

```
R3          O
|           ‖
C = CH - P - OR1        (II-N),
|           |
NH2        OR2
```

dans laquelle $R^1$, $R^2$ et $R^3$ sont comme définis ci-dessus;

ou (b) à faire réagir des composés de formules (IV), (III) et (II—N) dans un solvant organique.

5. Composition pharmaceutique contenant un 1,4-dihydropyridine-3-carboxylate selon la revendication 1; ou l'un de ses sels, en tant que principe actif pour utilisation dans le traitement de maladies de la circulation.

6. Composition pharmaceutique selon la revendication 5, pour utilisation dans le traitement de l'angine de poitrine.

7. Composition pharmaceutique selon la revendication 5, pour utilisation dans le traitement de l'hypertension.